# EUROPÄISCHE PATENTSCHRIFT

(11) **EP 1 135 401 B1**
(45) Veröffentlichungstag und Bekanntmachung des Hinweises auf die Patenterteilung: **18.06.2003**
(21) Anmeldenummer: 99958161.4
(22) Anmeldetag: 03.12.1999
(51) Int. Cl.: C07H 19/10, C07H 19/20, A61K 31/70

(54) **GLYCERYLNUCLEOTIDE, VERFAHREN ZU IHRER HERSTELLUNG UND IHRE VERWENDUNG**
GLYCERYL NUCLEOTIDES, METHOD FOR THE PRODUCTION THEREOF AND THEIR USE
GLYCERYLE-NUCLEOTIDES, LEUR PROCEDE DE PRODUCTION ET LEUR UTILISATION

(30) Priorität: 04.12.1998 DE 19855963
(43) Veröffentlichungstag der Anmeldung: 26.09.2001
(73) Patentinhaber: SCHOTT, Herbert, D-72076 Tübingen (DE)
(72) Erfinder: SCHOTT, Herbert, D-72076 Tübingen (DE); LUDWIG, Peter, Stephan, D-72764 Reutlingen (DE)
(74) Vertreter: Kinzebach, Werner, Dr.
(86) Internationale Anmeldenummer: EP9909461
(87) Internationale Veröffentlichungsnummer: WO00034298

(56) Entgegenhaltungen:
- EP-A- 0 457 570
- WO-A-93/16093
- WO-A-98/28978
- WO-A-98/38202

## Beschreibung

Die vorliegende Erfindung betrifft neue, gegebenenfalls amphiphile, Glycerylnucleotide, deren Herstellung sowie Mittel zur Behandlung von Krebserkrankungen und Infektionskrankheiten.

Nucleosidanaloga, die bestimmte Strukturmerkmale aufweisen, sind bewährte Arzneimittel in der Chemotherapie von Krebserkrankungen und virusbedingten Infektionskrankheiten (Advanced Drug Delivery Review (1996) 19, 287). Die therapeutische Wirkung der Nucleosidanaloga tritt allerdings nur dann auf, wenn die an sich unwirksamen Nucleosidanaloga als Prodrugs von der Zelle aufgenommen und dann zu den eigentlichen Wirkstoffen, den 5'-Triphosphatderivaten der Nucleosidanaloga anabolisiert werden. Diese Nucleotide stoppen die DNA-Replikation und/oder blockieren die Reverse-Transcriptase. Nucleosidanaloga wie beispielsweise 1-β-D-Arabinofuranosylcytosin (araC) und 5-Fluoro-2'-desoxyuridin (5FdU), die die DNA-Replikation verhindern, wirken gegen maligne Erkrankungen der blutbildenden Zellen und gegen solide Tumore. Zur Therapie der HIV-Infektion eignen sich besonders Didesoxynucleosidanaloga, wie beispielsweise 3'-Azido-2',3'didesoxythymidin (AZT), 2',3'-Didesoxycytidin (ddC), 2',3'-Didesoxyinosin (ddl), 3'-Thia-2',3'-didesoxycytidin (3TC) und 2',3'-Didehydro-2',3'-didesoxythymidin (d4T).

Ein nicht-nucleosidischer antiviraler Wirkstoff wie beispielsweise das Trinatriumsalz der Phosphonoameisensäure (Foscarnet) blockiert die Pyrophosphat-Bindungsseite der viralen Polymerase. Hierdurch wird die Replikation beispielsweise bei Herpes Simplex-, HIV- und Human Cytomegaloviren verhindert. Amphiphile Glycerylderivate von Foscarnet, die eine verbesserte Membranpassage aufweisen, tragen zur Optimierung der Virustherapie bei (Antivir. Chem. & Chemother. (1998) 9, 33).

Aufgrund der Resistenzbildung im Verlauf der Chemotherapie, die vor allem bei der HIV-Behandlung besonders schnell auftritt, kann die Progression der Erkrankung nur durch eine Kombinationstherapie nachhaltig verlangsamt werden. Hierbei werden mehrere antivirale Wirkstoffe gemeinsam appliziert (Schweiz. Med. Wochenschr. (1997) 127, 436). Durch das strenge Therapie-Reglement, das den Patienten bei der Kombinationstherapie auferlegt werden muß, ist die Compliance der Patienten niedrig. Der therapeutische Erfolg liegt daher weit unter den Möglichkeiten, die das hohe Potential der verfügbaren Arzneimittel in sich birgt. (AIDS 1998 Diagnostik und Therapie, Steinhauser Verlag).

Die Applikation einer Darreichungsform in der beispielsweise die zwei nucleosidischen Prodrugs (AZT, 3TC) als Gemisch vorliegen, wie dies beim Combivir der Fall ist, macht die Kombinationstherapie für den Patienten allenfalls nur praktikabler. Eine verbesserte Wirkung ist aber mit solchen Gemischen kaum erreichbar, da weder die Zellaufnahme der Prodrugs gesteigert, noch deren Anabolisierung zum Wirkstoff optimiert wird.

Dagegen kann man mit amphiphilen Kombinationspräparaten, in denen zwei antivirale Nucleosidanaloga chemisch über eine Phosphodiesterbindung gekuppelt sind, die Kombinationstherapie entscheidend optimieren (EP 0 642 527 B 1). Ein gewisser Nachteil dieser amphiphilen Dinucleosidphosphatanaloga besteht darin, daß bei einer erwünschten enzymatischen Spaltung der Phosphodiesterbindung jeweils nur eine Monomereinheit als wirksames Nucleotidanaloga freigesetzt werden kann, während die zweite Monomereinheit des Kombinationspräparats zwangsläufig als an sich unwirksames Nucleosidanaloga verbleibt. Falls die Zelle dieses Nucleosidanaloga nicht zum wirksamen Nucleotidanaloga anabolisiert, sind bis zu 50 % des aplizierten Dimeren therapeutisch nicht nutzbar und somit unwirksam. Ein zusätzlicher Nachteil dieser amphiphilen Kombinationspräparate liegt darin, daß mindestens eins der beiden gekuppelten Nucleosidanaloga einen lipophilen Rest aufweisen muß, damit das resultierende Dimere amphiphil ist. Zwei zur Kombinationstherapie geeignete Nucleosidanaloga, die beide nicht lipophilisierbar sind, können somit nicht in amphiphile Dimere überführt und als Kombinationspräparat in der Therapie genutzt werden.

Aufgabe dieser Erfindung ist es, neue Kombinationspräparate bereitzustellen, mit denen Krebserkrankungen und Infektionen noch wirksamer bekämpft werden können. Die Aufgabe wird durch neue Glycerylnucleotide gelöst, die bei der Metabolisierung jeweils zwei Wirkstoffe gleichzeitig freisetzen können, so daß die Vorteile der genannten Kombination zweier Wirkstoffe uneingeschränkt zum Tragen kommen. Zur Darstellung der erfindungsgemäßen Glycerylnucleotide werden vorzugsweise entweder zwei therapeutisch wirksame Nucleosidderivate miteinander oder ein Nucleosidderivat mit der Phosphonoameisensäure oder ihrer Salzform (Foscarnet) über ein Glycerinlipidgerüst kovalent verbunden.

Ein erster Gegenstand der Erfindung betrifft Glycerylnucleotide der Formel la 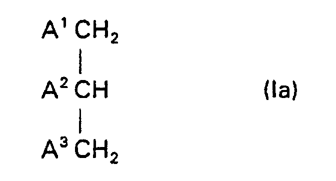 worin
a) einer der Reste A¹, A² und A³ für ein Wasserstoffatom, oder einen Rest, ausgewählt unter Hydroxyl, Mecapto, Alkyl, Alkenyl, Polyoxyalkenyl, Aryl, Acyl, Alkyloxy, Alkenyloxy, Polyoxyalkenyloxy, Acyloxy, Aryloxy, Alkylthio, Alkenylthio, Acylthio oder Arylthio stehen, wobei die Alkyl-, Alkenyl- und Acyl gegebenenfalls mit 1 bis 3 Aryl- Resten substituiert sind; und
b1) zwei der verbleibenden Reste A¹, A² und A³ für zwei voneinander verschiedene Nucleosidgruppen stehen, welche jeweils über eine physiologisch spaltbare phosphorhaltige Brückengruppe mit dem Kohlenstoffatom der Glycerylkette verbunden sind; oder
b2) einer der verbleibenden Reste A¹, A² und A³ für eine Nucleosidgruppe und der andere der verbleibenden Reste für eine Hydroxycarbonylgruppe steht, welche jeweils über eine physiologisch spaltbare phosphorhaltige Brückengruppe mit dem Kohlenstoffatom der Glycerylkette verbunden sind;
wobei wenigstens eine der Nucleosidgruppen keine natürlich vorkommende Nucleosidgruppe ist, welche in ihrem Basenteil, an einem oder mehreren Ringatomen und/oder an einer oder mehreren Seitengruppen, wie z.B. Amino-Seitengruppen, gegebenenfalls substituiert ist mit einem oder mehreren Resten, ausgewählt unter Hydroxyl, Amino, Halogen, Alkyl, Alkenyl, Polyoxyalkenyl, Aryl, Acyl, Alkyloxy, Alkenyloxy, Polyoxyalkenyloxy, Acyloxy, Aryloxy, Alkylthio, Alkenylthio, Acylthio oder Arylthio, wobei die Alkyl-, Alkenyl- und Acyl gegebenenfalls mit 1 bis 3 Aryl- Resten oder Halogenatomen substituiert sind; und welche in ihrem Kohlenhydratteil gegebenenfalls ein- oder mehrfach substituiert ist mit Substituenten, ausgewählt unter Wasserstoff, Halogen, wie F, Cl, Br und J, Hydroxyl, Ethinyl oder Azido, gegebenenfalls ein Heteroatom, ausgewählt unter S , N und O anstelle eines Kohlenstoffatoms aufweist und gegebenenfalls eine oder zwei nichtbenachbarte C = C-Doppelbindungen enthält;
in racemischer oder enantiomerenreiner Form sowie die pharmazeutisch verträglichen Salze dieser Verbindungen.

Die in den erfindungsgemäßen Verbindungen enthaltenen nicht natürlich vorkommenden Nucleosidgruppen sind abgeleitet von Nucleosiden (Nucleosidderivaten), umfassend einen heterocyclischen Rest (Basenteil), welcher N- oder O-glycosidisch mit einem Zuckerrest (Kohlenhydratteil) verbunden ist. Sie unterscheiden sich von den natürlich vorkommenden Nucleosiden Adenosin, Guanosin, Cytidin, Uridin und Thymidin und den entsprechenden Desoxynucleosiden im Kohlehydrat- und/oder im Basenteil.

Der Zuckerrest des nicht natürlich vorkommenden Nucleosids oder Nucleosidderivats ist abgeleitet von einer Hexose oder Heptose, vorzugsweise von einer Pentose, wie z.B. Desoxyribose oder Ribose. Im Zuckerrest können gegebenenfalls einzelne oder mehrere Protonen oder Hydroxylgruppen substituiert oder eliminiert sein. Geeignete Substituenten sind dabei ausgewählt unter den oben angegebenen Substituenten Wasserstoff, Halogen, wie F, Cl, Br und J, Hydroxyl, Ethinyl und Azido. Gegebenenfalls kann ein Heteroatom, ausgewählt unter S , N und O anstelle eines Kohlenstoffatoms enthalten sein und gegebenenfalls kann der Zuckerrest eine oder zwei nichtbenachbarte C = C-Doppelbindungen enthalten.

Der Basenteil des nicht natürlich vorkommenden Nucleosids oder Nucleosidderivats ist der Rest einer ein- oder zweikernigen heterocyclische Base, zusammengesetzt aus einem oder zwei vier- bis siebengliedrigen Ringen, welche zusammen wenigstens ein Ring-Heteroatom, wie z.B. ein bis sechs Heteroatome, ausgewählt unter N, S und O, insbesondere N und O, enthalten. Beispiele für derartige Basen sind die Purin- und Pyrimidin-Basen Adenin, Guanin, Cytosin, Uracil und Thymin. Weitere Beispiele für brauchbare Basen sind Pyrrol, Pyrazol, Imidazol, Aminopyrazol, 1,2,3-Triazol, 1,2,4-Triazol, Tetrazol, Pentazol, Pyridon, Piperidin, Pyridin, Indol, Isoindol, Pyridazin, Indoxyl, Isatin, Pyrazin, Piperazin, Gramin, Tryptophan, Kymurensäure, Tryptamin, 3-Indolylessigsäure, Carbazol, Indazol, 1,3,5-Triazin, 1,2,4-Triazin, 1,2,3-Triazin und Tetrazin. Bevorzugte Basen sind Adenin, Guanin, Cytosin, Uracil und Thymin; sowie 1,2,3-Triazol, 1,2,4-Triazol und Tetrazol. Die genannten Basen können gegebenenfalls ein- oder mehrfach, wie z.B. ein- bis vierfach, insbesondere ein- oder zweifach substituiert sein durch die oben angegebenen Reste Hydroxyl, Amino, Halogen, Alkyl, Alkenyl, Polyoxyalkenyl, Aryl, Acyl, Alkyloxy, Alkenyloxy, Polyoxyalkenyloxy, Acyloxy, Aryloxy, Alkylthio, Alkenylthio, Acylthio oder Arylthio, wobei die Alkyl-, Alkenyl- und Acyl gegebenenfalls mit 1 bis 3 Aryl- Resten oder Halogenatomen substituiert sind. Dabei kann die Substitution an einem Ring-Heteroatom oder bevorzugt an einem Ring-Kohlenstoffatom oder einer Seitengruppe, wie z.B. einer Amino-Seitengruppe der Base erfolgen.

In den erfindungsgemäßen Verbindungen der Formel (la) sind die physiologisch spaltbaren phosphorhaltigen Brückengruppen vorzugsweise von Phosphodiestergruppen und deren schwefelhaltigen Analoga abgeleitet. In einer bevorzugten Ausführungsform werden daher solche Verbindungen der Formel la bereitgestellt, worin Nucleosidgruppen unabhängig voneinander über eine Brückengruppe ausgewählt unter -OP(OZ)(O)O-, -SP(OZ)(O)O-, -OP(OZ)(S)O- oder -SP(OZ)(S)O- und die Hydroxycarbonylgruppe über eine Brückengruppe ausgewählt unter -OP(OZ)(O)-, -SP(OZ)(O)- oder -SP(OZ)(S)-, worin Z für ein Proton oder ein pharmazeutisch vertägliches Kation steht, mit dem Glycerylrest verbunden sind.

Besonders bevorzugt sind solche Verbindungen der Formel la worin A¹, A² und A³ so gewählt sind, daß dem Molekül ein amphiphiler Charakter verliehen wird. Dies erreicht man dadurch, daß man den Glycerylrest oder den (die) Nucleosidderivat-Rest(e) mit einem lipophilen Substituenten substituiert. Der Nucleosidderivat-Rest trägt dabei den lipophilen Rest bevorzugt am Basenteil. Als Beispiele für solche lipophilen Reste sind zu nennen Alkyl, Alkenyl, Polyoxyalkenyl, Aryl, Acyl, Alkyloxy, Alkenyloxy, Polyoxyalkenyloxy, Acyloxy, Aryloxy, Alkylthio, Alkenylthio, Acylthio oder Arylthio, wobei die Alkyl-, Alkenyl- und Acyl gegebenenfalls mit 1 bis 3 Aryl- Resten oder Halogenatomen substituiert sind.

Der lipophile Rest sollte vorzugsweise mehr als 6, wie z.B. 7 bis 30 oder 10 bis 24 Kohlenstoffatome umfassen.

Als Beispiele für geeignete Aryl-Reste sind zu nennen: Phenyl, Naphthyl, und Benzyl.

Als Beispiele für geeignete Alkylreste sind zu nennen geradkettige oder verzweigte Reste mit 1 bis 24 C-Atomen, wie Methyl, Ethyl, i- oder n-Propyl, n-, i-, sec.- oder tert.-Butyl, n- oder i-Pentyl; außerdem n-Hexyl, n-Heptyl, n-Octyl, n-Nonyl, n-Decyl, n-Undecyl, n-Tridecyl, n-Tetradecyl, n-Pentadecyl und n-Hexadecyl, Octadecyl, Docosanyl sowie die ein- oder mehrfach verzweigten Analoga davon.

Als Beispiele für geeignete Alkenylreste sind die ein- oder mehrfach, vorzugsweise einfach oder zweifach ungesättigten Analoga oben genannter Alkylreste mit 2 bis 24 Kohlenstoffatomen, wobei die Doppelbindung in beliebiger Position der Kohlenstoffkette liegen kann.

Beispiele für geeignete Polyoxyalkenylreste sind abgeleitet von C₂-C₄-Alkylenoxiden, welche 2 bis 12 wiederkehrende Alkyenoxideinheiten umfassen können.

Beispiele für geeignete Acylreste sind abgeleitet von geradkettigen oder verzweigten, gegebenenfalls ein- oder mehrfach ungesättigten, gegebenenfalls substituierten C₁-C₂₄-Monocarbonsäuren. Beispielsweise sind brauchbare Acyl reste abgeleitet von folgenden Carbonsäuren: Gesättigten Säuren , wie Ameisen-, Essig-, Propion- und n- und i-Buttersäure, n- und i-Valeriansäure, Capronsäure, Önanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Behensäure, Lignocerinsäure, Cerotinsäure und Melissinsäure; einfach ungesättigte Säuren, wie Acrylsäure, Crotonsäure, Palmitoleinsäure, Ölsäure und Erucasäure; und zweifach ungesättigten Säuren, wie Sorbinsäure und Linolsäure. Sind in den Fettsäuren Doppelbindungen enthalten, so können diese sowohl in cis- als auch in trans-Form vorliegen.

Beispiele für geeignete Alkyloxy-, Acyloxy,- Aryloxy- Alkenyloxy und Polyoxyalkylenoxyreste sind die Sauerstoff-terminierten Analoga oben genannter Alkyl-, Acyl-, Aryl-, Alkenyl und Polyoxyalkylenreste.

Beispiele für geeignete Alkylthio, Alkenylthio, Acylthio oder Arylthio sind die entsprechenden Schwefel-terminierten Analoga obiger Alkyloxy-, Alkenyloxy-Acyloxy- und Aryloxy-Reste.

Gegenstand der Erfindung sind insbesondere, vorzugsweise amphiphile, Glycerylnucleotide der Formel I, 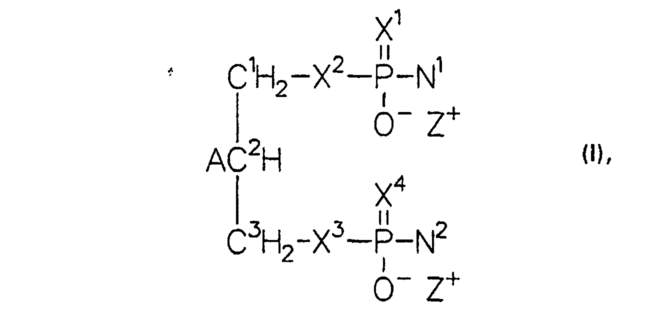 in racemischer und enantiomeren reiner Form, worin der Rest A und die beiden Phosphorsäurereste in beliebiger Reihenfolge mit den C-Atomen C¹, C² und C³ des Glyceringerüstes verbunden sein können;
X¹, X², X³ und X⁴ gleich oder verschieden sind und Sauerstoff und Schwefel bedeuten;
A für Alkyl, Hydroxyl, Thiol oder eine Alkoxy-, Alkylthio-, Alkylcarboxy- oder Alkylthiocarboxygruppe steht, wobei die Alkylreste linear oder verzweigt sind, 1-24 C-Atome und bis zu 2 Doppelbindungen aufweisen und durch 1 bis 3 aromatische Reste substituiert sein können;
Z für Wasserstoff oder das entsprechende Salz der sauren Form dieser Verbindung; N¹ oder N² für Hydroxycarbonyl oder dessen Salzform steht;und der andere der Reste N¹ und N² ein D- oder L-konfiguriertes Nucleosidderivat der Formel II, III und IV bedeutet, und beide Reste N¹ und N² verschieden sind, wenn sie beide für ein D-oder L-konfiguriertes Nucleosidderivat der Formel II, III, und IV, 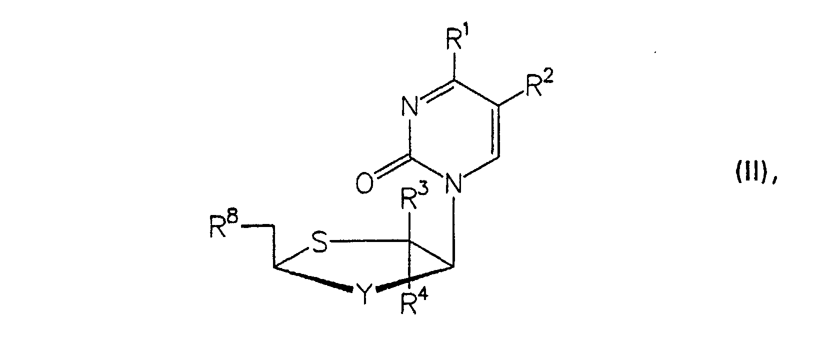 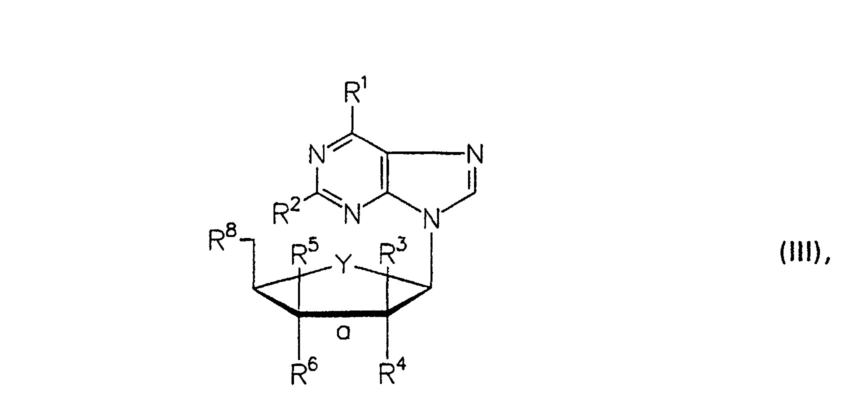 oder 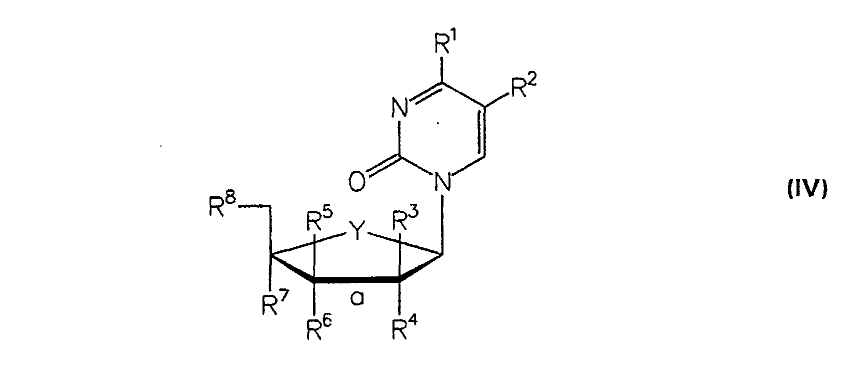 stehen, wobei
Y für Sauerstoff oder Schwefel steht;
R¹ eine Hydroxyl-, Amino-, acylierte, alkylierte oder durch Polyoxyethylen substituierte Aminogruppe bedeutet, deren Acyl- oder Alkylrest linear oder verzweigt ist, 1-24 C-Atome und bis zu 2 Doppelbindungen aufweist und durch einen aromatischen Rest substituiert sein kann;
R² für Wasserstoff, Halogen, eine Amino- oder Hydroxylgruppe, einen Bromvinyl-, einen linearen oder verzweigten C₁ bis C₂₄ Alkylrest steht;
R³, R⁴, R⁵, R⁶, R⁷ und R⁸ gleich oder verschieden sind und für Wasserstoff, Halogen, Hydroxyl, Ethinyl und Azido stehen;
und einer der Rest R³ bis R⁸ Sauerstoff bedeutet über den das Nucleosidderivat mit dem Glycerylphosphat verbunden ist, und zwei der Reste R³ bis R⁶ dann entfallen, wenn a eine C = C-Doppelbindung darstellt.

Vorzugsweise sind die Reste A, R¹ und R² in Formel I so gewählt, daß man eine Verbindung mit amphiphilem Charakter erhält.

Ist A ein Alkoxyrest, so sind Reste mit 12 bis 24 C-Atomen, wie der Hexadecyloxy-, Octadecyloxy- oder Docosanyloxyrest, bevorzugt.

Ist A ein Carbonsäurerest, so sind Reste mit 12 bis 24 C-Atomen, wie der Palmitin-, Stearin- oder Behensäurerest, bevorzugt.

Ist A ein Alkylrest, so sind Reste mit 12 bis 24 C-Atomen, wie der Hexadecyl-, 9-Oktadecenyl-, Oktadecyl-, oder Docosanylrest bevorzugt.

Ist R¹ eine alkylierte Aminogruppe, so ist deren Alkylrest vorzugsweise ein Rest mit 12 bis 24 C-Atomen, wie ein Hexadecyl- oder Octadecylrest; ist R¹ eine acylierte Aminogruppe, so ist der Acylrest bevorzugt ein Rest mit 12 bis 24 C-Atomen , wie ein Palmitoyl, Oleoyl- und Behenoylrest.

R² steht vorzugsweise für Wasserstoff, Halogen, Methyl oder Ethyl; R³, R⁴, R⁵, R⁶ und R⁷ stehen bevorzugt für Azido, Wasserstoff, Fluor, Ethinyl oder Hydroxyl; und R⁸ ist eine Hydroxylgruppe bevorzugt. Insbesondere bevorzugt steht R⁸ jedoch für ein Sauerstoffatom über welches der Nucleosidrest an das P-Atom der Brückengruppe gebunden ist.

Ist einer der beiden Reste N¹ oder N² ein Hydroxycarbonylrest, dann ist für den anderen Rest ein Nucleosidderivat der Formel II oder IV bevorzugt.

Weitere bevorzugte Gruppen von Verbindungen sind:
a) Verbindungen der Formel (I), worin
   X¹, X², X³ und X⁴ ein Sauerstoffatom bedeuten;
   das C¹-Atom des Glyceringerüstes der Formel I mit dem Rest A verbunden ist, wobei A für Hydroxyl, Octadecyl, Octadecyloxy, Docosyloxy oder Behenoyloxy, Palmitoyl oder Oleoyl steht;
   das C²-Atom des Glyceringerüstes der Formel I über eine Phosphodiesterbrücke mit N² verbunden ist, wobei N² für einen Nucleosidderivat-Rest der Formel II, III oder IV steht, worin
   Y für ein Sauerstoffatom steht;
   R¹ für eine Hydroxyl-, Amino-, Octadecylamino-, Docosylamino-, Palmitoylamino-, Oleoylamino- oder Behenoylaminogruppe steht;
   R² für Methyl, Ethyl, Wasserstoff oder Halogen steht;
   R³ bis R⁸ die oben angegebenen Bedeutungn besitzen, wobei einer der Reste R³, R⁴, R⁵, R⁶ und R⁸ ein Sauerstoffatom darstellt, über das der Nucleosidderivat-Rest N² mit dem Phosphoratom verbunden ist,
   und
   das C³-Atom des Glyceringerüstes der Formel I mit dem Hydroxycarbonylphosphonatrest in seiner freien oder Salzform verbunden ist.
b) Verbindungen der Formel I gemäß Gruppe a), worin insbesondere
   N² für einen Nucleosidderivatrest der Formel II oder IV steht, worin
   R¹ eine Amino-, Palmitoylamino- oder Hydroxylgruppe bedeutet;
   R² Wasserstoff, Methyl oder Ethyl bedeutet;
   R³, R⁴, R⁵ und R⁷ für Wasserstoff stehen;
   R⁶ Wasserstoff, Fluor oder Azido bedeuten, und
   R⁸ ein Sauerstoffatom darstellt, über das N² mit dem Phosphoratom verbunden ist.
c) Verbindungen der Formel I, worin
   X¹, X², X³ und X⁴ ein Sauerstoffatom bedeuten;
   A für Palmitoyloxy-, Oleoyoxyl- und Octadecyloxy steht und mit dem C²-Atom des Glyceringerüstes der Formel I verbunden ist und
   N¹ und N² verschieden sind und für einen Nucleosidderivat-Rest der Formeln II, III und IV stehen.
d) Verbindungen der Formel I gemäß Gruppe c, worin insbesondere
   N¹ für einen, vorzugsweise L-konfigurierten, Nucleosidderivat-Rest der Formel II steht,
   worin
   R¹ eine Amino- oder Palmitoylaminogruppe bedeutet;
   R², R³ und R⁴ für Wasserstoff stehen, und
   R⁸ ein Sauerstoffatom darstellt, über das N¹ mit dem Phosphoratom in Position C¹ oder C³ des Glyceryldiphosphatgerüstes der Formel I verbunden ist; und N² für einen Nucleosidderivat-Rest der Formel III oder IV steht.
e) Verbindungen der Formel I gemäß Gruppe c, worin insbesondere
   N² einen Nucleosidderivat-Rest der Formel IV darstellt, worin
   R¹ für eine Amino-, Palmitoylamino- oder Hydroxylgruppe steht;
   R² für Wasserstoff oder Methyl steht;
   R³, R⁴, R⁵ und R⁷ für Wasserstoff stehen;
   R⁶ für Wasserstoff, Fluor oder Azido steht, und
   R⁸ für ein Sauerstoffatom steht, über das N² mit dem Phosphoratom in Position C¹ oder C³ des Glyceryldiphosphatgerüstes der Formel I verbunden ist.
f) Verbindungen der Formel I gemäß Gruppe c, worin insbesondere
   N¹ für einen Nucleosidderivat-Rest der Formel II oder IV steht, und
   N² für einen Nucleosidderivat-Rest der Formel III steht,
   worin jeweils
   R¹ für Hydroxyl steht;
   R² bis R⁷ für Wasserstoff stehen; und
   R⁸ für ein Sauerstoffatom steht, über das N¹ bzw. N² mit dem Glycerin-1,3-diphosphatgerüst der Formel I verbunden ist.
g) Verbindungen der Formel I gemäß Gruppe c, worin insbesondere
   N¹ und N² für einen Nucleosidderivat-Rest der Formel IV stehen, worin in N¹
   R¹ für eine Amino-, Palmitoylamino- oder eine Octadecylaminogruppe steht;
   R², R⁵ und R⁷ für Wasserstoff stehen;
   R³, R⁴ und R⁶ gleich oder verschieden sind und für Wasserstoff, Hydroxyl oder Fluor stehen;
   und worin in N²
   R¹ für eine Hydroxylgruppe steht;
   R² für Methyl oder Halogen steht;
   R³, R⁴, R⁵ und R⁷ für Wasserstoff stehen, wobei gegebenenfalls zwei vicinale Reste davon entfallen, wenn in Position a eine Doppelbindung vorliegt;
   R⁶ für Wasserstoff, Fluor, Azido oder Hydroxyl stehen; und
   R⁸ in N¹ und N² jeweils ein Sauerstoffatom darstellt, über das N¹ und N² mit dem Glycerin-1,3-diphosphatgerüst verbunden sind.
h) Verbindungen der Formel I gemäß Gruppe g, worin insbesondere
   in N¹ und N²
   R¹ gleich oder verschieden ist und für eine Hydroxyl- oder Aminogruppe steht;
   R² gleich oder verschieden ist und für Wassserstoff oder Methyl steht;
   R⁴, R⁵ und R⁷ für Wasserstoff stehen;
   R⁶ in N¹ für Azido und in N² für Fluor steht; und
   R⁸ das Sauerstoffatom bedeutet, über das N¹ und N² mit dem Glycerin-1,3-diphosphatgerüst verbunden ist.
i) Verbindungen der Formel I gemäß Gruppe g, worin insbesondere,
   in N¹,
   R¹ für eine Amino- der Palmitoylaminogruppe steht,
   R² und R⁷ für Wasserstoff stehen,
   R³ und R⁴ für Fluor, Wasserstoff oder Hydroxyl stehen,
   R⁵ für Wasserstoff oder einen Ethinylrest steht,
   R⁶ für Hydroxyl steht, und
   worin in N²,
   R¹ für Hydroxyl steht,
   R² für Fluor steht,
   R³, R⁴, R⁵ und R⁷ für Wasserstoff stehen;
   R⁶ für Hydroxyl steht, und
   R⁸ in N¹ und N² jeweils ein Sauerstoffatom darstellt, über N¹ und N² mit dem Glycerin-1,3-diphosphatgerüst verbunden sind.

Die neuen, vorzugsweise amphiphilen, Glycerylnucleotide der Formel la lassen sich herstellen, indem man eine Verbindung der Formel lb 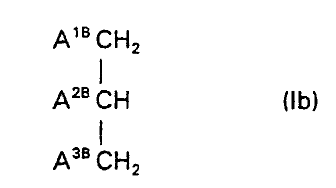 worin
einer der Reste A^{1B}, A^{2B} und A^{3B} für eine Hydroxy-, Mercapto-, Hydrogenphosphonat- oder Thiohydrogenphosphonatgruppe steht, und die beiden anderen Reste die oben für A¹, A² und A³ angegebenen Bedeutungen besitzen, wobei wenigstens einer der beiden Reste für eine Nucleosidgruppe gemäß obiger Definition steht,
a) in Gegenwart eines Säurechlorids mit einem Nucleosid oder Nukleosidderivat gemäß oben angegebener Definition kondensiert, wobei das Nukleosidderivat zusätzlich eine Hydrogenphosphonat- oder Thiohydrogenphosphonat-Gruppe trägt, wenn einer der Reste A^{1B}, A^{2B} und A^{3B} für eine Hydroxy-, oder Mercapto-Gruppe steht; und das erhaltenen Produkt oxidiert; oder
b) mit (Ethoxycarbonyl)dichlorophosphonat umsetzt und anschließend die Säurechlorid- und Ethoxygruppen alkalisch hydrolysiert.

Die erfindungsgemäßen, vorzugsweise amphiphilen, Glycerylnucleotide der Formel I, in der N¹ und N² für ein D- oder L-konfiguriertes Nucleosidderivat stehen, lassen sich herstellen, in dem man eine Verbindung der Formel V, 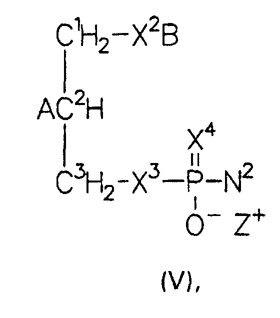 worin die Reste A, X²B und der Phosphorsäurerest in beliebiger Reihenfolge mit den C¹-, C²- und C³-Atomen des Glyceringerüstes verbunden sein können; die Reste A, X², X³, X⁴, N² und Z die angegebene Bedeutungen haben; und X²B für eine Hydroxy-, Thiol-, Hydrogenphosphonat- oder Thiohydrogenphosphonatgruppe steht;
mit einem Nucleosidderivat obiger Formel II, III oder IV, worin die Reste R¹ bis R⁸ die angegebenen Bedeutungen haben, und zusätzlich R⁸ auch für 4-Mono-, 4,4'-Dimethoxytriphenylmethoxy, Hydrogenphosphonat oder Thiohydrogenphosphonat stehen kann; R³, R⁴, R⁵ und R⁶ zusätztlich für einen linearen oder verzweigten Carboxylrest stehen kann, der 1 - 24 C-Atome aufweist und durch einen Phenylrest substituiert sein kann; und immer einer der Reste R³, R⁴, R⁵, R⁶ und R⁸ für Hydrogenphosphonat oder Thiohydrogenphosphonat steht, wenn X²B in einer Verbindung der Formel V für Hydroxyl oder Thiol steht, dagegen aber keiner dieser Reste Hydrogenphosphonat oder Thiohydrogenphosphonat bedeutet, wenn X²B für Hydrogenphosphonat oder Thiohydrogenphosphonat steht;
in Gegenwart eines Säurechlorids kondensiert und anschließend oxidiert.
Die Kondensation gelingt besonders gut in Gegenwart von Säureanhydriden oder Säurehalogeniden, wie insbesondere Pivalinsäurechlorid, bei -80 °C bis + 100°C, wie z.B. etwa 0 - 20°C. Die Oxidation gelingt besonders gut bei -80 °C bis + 100 °C, wie z.B. etwa 0 - 20°C, wobei, a) mit Jod in wässrigen organischen Lösungsmitteln die P-H-Bindung zu einer P=O-Bindung oder b) mit S₈ in Triethylamin/CS₂ die P-H-Bindung zu einer P=S-Bindung oxidiert wird.

Nach Oxidation und chromatographischer Aufarbeitung wird die 4-Mono- oder 4,4'-Dimethoxytriphenylmethylgruppe gegen Hydroxyl getauscht. Acylreste werden im Bedarfsfall hydrolytisch in Mercapto-, Hydroxyl- und/oder Aminogruppen überführt.

Die neuen, vorzugsweise amphiphilen, Verbindungen der Formel I, in der N¹ ein Hydroxycarbonylrest bedeutet, der in seiner Säure- oder Salzform gebunden ist, kann man herstellen, in dem eine Verbindung der Formel V, in der B für Wasserstoff steht, mit (Ethoxycarbonyl)phosphorsäuredichlorid in an sich bekannter Weise umgesetzt wird. Die Umsetzung gelingt besonders gut in Gemischen mit halogenierten Kohlenwasserstoffen, wie insbesondere Pyridin/Chloroform, Acetonitril/Chloroform oder Pyridin/Methylenchlorid und Acetonitril/Methylenchlorid, und Phosphorsäuretrimethylester bei -10 °C bis + 80°C, wie z.B. etwa 0 - 10°C. Nach der Umsetzung und chromatographischen Aufarbeitung werden durch anschließende alkalische Hydrolyse die Säurechlorid- und Ethoxygruppe in Hydroxylgruppen oder entsprechende Salzformen getauscht (J. Med. Chem. (1986) 29, 1389).

Die als Ausgangsmaterial verwendeten Verbindungen der Formel V lassen sich durch Kondensation einer Verbindung der Formel VI 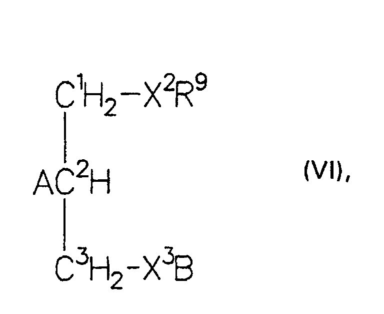 worin die Reste
A, X²R⁹ und X³B in beliebiger Reihenfolge mit den C¹-, C²- und C³-Atomen des Glyceringerüstes verbunden sein können;
A und X² die oben angegebenen Bedeutungen haben;
R⁹ für 4-Mono-, 4,4'-Dimethoxytriphenylmethyl oder einen linearen oder verzweigten Acylrest, der 1 - 24 C-Atome aufweist und durch einen aromatischen Rest substituiert sein kann, steht;
der Rest X³B für für eine Hydroxy-, Thiol-, Hydrogenphosphonat- oder Thiohydrogenphosphonatgruppe steht;
mit einem Nucleosidderivat der Formel II, III oder IV, worin die Reste R¹ - R⁸ die oben angegebenen Bedeutungen haben,
wobei immer einer der Reste R³, R⁴, R⁵, R⁶ und R⁸ auch für Hydrogenphosphonat oder Thiohydrogenphosphonat steht, wenn der Rest X³B in einer Verbindung der Formel VI für Hydroxyl oder Thiol steht, dagegen aber keiner der Reste Hydrogenphosphonat oder Thiohydrogenphosphonat bedeutet, wenn X³B in einer Verbindung der Formel VI für Hydrogenphosphonat oder Thiohydrogenphosphonat steht;
in Gegenwart eines Säurechlorids und anschließender Oxidation mit Jod oder Schwefel in an sich bekannter Weise herstellen. (Tetrahedron Lett, (1986) 27, 469; ibid 5575).

Nach der chromatographischen Aufarbeitung wird der 4-Mono- oder 4',4'-Dimethoxytriphenylmethylrest unter sauren Bedingungen, der Acylrest unter alkalischen Bedingungen gegen Wasserstoff ausgetauscht.

Die für die Umsetzungen benötigten Ausgangsmaterialien sind bekannte Stoffe oder lassen sich in Analogie zu bekannten Verfahren herstellen (Hel. Chim. Acta (1982) 65, 1059; Liebigs Ann. Chem. (1991) 765; ibid (1996) 365; Antivir. Chem & Chemother. (1998) 9, 33. J.C.S. Perkin I (1982) 11 71; Makromol Chem. (1986) 187, 809; Tetrahedron Lett. (1986) 27, 2661).

Die erfindungsgemäßen Verbindungen können sowohl amphiphilen als auch nichtamphiphilen Charakter besitzen. Besonders bevorzugt sind jedoch amphiphile Verbindungen.

Die erfindungsgemäße Überführung von Foscarnet und der therapeutisch wirksamen Nucleosidanaloga in amphiphile Glycerylnucleotide bedingt ein stark verändertes pharmakokinetisches Verhalten. Hierdurch kann die zu applizierende Dosis im Vergleich zum jeweiligen Monomer überraschenderweise erhöht werden, ohne daß es gleichzeitig zu einer Verstärkung aller toxischen Nebeneffekte dieser hochpotenten Medikamente führt. Außerdem wirken amphiphile Glycerylnucleotide auch noch bei Resistenz gegen die jeweiligen monomeren Nucleosidanaloga.

Der amphiphile Charakter erfindungsgemäßer amphiphilere Glycerylnucleotide ermöglicht unterschiedliche Applikationsschemata. Der lipophile Bereich der Glycerylnucleotide bewirkt, daß die Wirkstoffe zusammen mit Matrixlipiden stabil in Liposomen eingebaut werden, begünstigt die Zellaufnahme der in Wasser gelösten amphiphilen Glycerylnucleotide und schützt sie gleichzeitig vor einer zu schnellen enzymatischen Hydrolyse. Der hydrophile Bereich ermöglicht, daß die Wirkstoffe vermutlich über eine Micellenbildung auch wasserlöslich sind. Die alternative Applikationsmöglichkeit der amphiphilen Glycerylnucleotide hat den Vorteil, daß die gewünschte Depotwirkung der Dimeren nicht nur in einer Liposomendispersion sondern auch in wässrigen Lösungen erreicht wird, so daß man nicht ausschließlich auf die Liposomentechnologie angewiesen ist, sie aber bei Bedarf nutzen kann, was wiederum bei nur wasserlöslichen Wirkstoffen nicht problemlos möglich ist.

Ein weiterer Vorteil der erfindungsgemäßen amphiphilen Glycerylnucleotide liegt darin, daß sie zusammen mit einem oder mehreren anderen Wirkstoffen in unterschiedlichen Mengen in Liposomen eingebaut werden können, wobei synergistische Wirkungen resultieren. Mit diesen amphiphilen Glycerylnucleotiden und/oder in Zusammensetzung mit einem biologisch verträglichen Träger und/oder mit Mitteln, daß die erfindungsgemäßen Verbindungen mindestens in einer oder mehreren der Zusammensetzungen enthalten, kann die Therapie von Krebserkrankungen und virusbedingten Infektionskrankheiten optimiert werden.

Die erfindungsgemäßen Verbindungen werden im allgemeinen in Form pharmazeutischer Mittel zur Behandlung eines Individuums, vorzugsweise eines Säugers, insbesondere eines Menschen, eingesetzt. So werden die Verbindungen gewöhnlich in Form von pharmazeutischen Zusammensetzungen verabreicht, die einen pharmazeutisch verträglichen Exzipienten mit wenigstens einem erfindungsgemäßen Nucleosidphosphatanalogon, gegebenfalls auch ein Gemisch mehrerer erfindungsgemäßer Verbindungen, und gegebenenfalls weitere für den jeweiligen gewünschten therapeutischen Effekt brauchbare Wirkstoffe umfassen. Diese Zusammensetzungen können beispielsweise auf oralem, rektalem, transdermalem, subkutanem, intravenösem, intramuskulärem oder intranasalem Weg verabreicht werden.

Beispiele geeigneter pharmazeutischer Formulierungen sind feste Arzneiformen, wie Pulver, Puder, Granulate, Tabletten, Pastillen, Sachets, Cachets, Dragees, Kapseln wie Hart- und Weichgelatinekapseln, Suppositorien oder vaginale Arzneiformen; halbfeste Arzneiformen, wie Salben, Cremes, Hydrogele, Pasten oder Pflaster, sowie flüssige Arzneiformen, wie Lösungen, Emulsionen, insbesondere Ölin-Wasser-Emulsionen, Suspensionen, beispielsweise Lotionen, Injektions- und Infusionszubereitungen, Augen- und Ohrentropfen. Auch implantierte Abgabevorrichtungen können zur Verabreichung erfindungsgemäßer Verbindungen verwendet werden. Ferner können auch Liposomen, Mikrosphären oder Polymermatrizes zur Anwendung kommen.

Bei der Herstellung der Zusammensetzungen werden erfindungsgemäße Verbindungen gewöhnlich mit einem Exzipienten vermischt oder verdünnt. Exzipienten können feste, halbfeste oder flüssige Materialien sein, die als Vehikel, Träger oder Medium für den Wirkstoff dienen.

Zu geeigneten Exzipienten gehören beispielsweise Lactose, Dextrose, Succrose, Sorbitol, Manitol, Stärken, Akaziengummi, Calciumphosphat, Alginate, Traganth, Gelantine, Calciumsilikat, mikrokristalline Cellulose, Polyvinylpyrrolidon, Cellulose, Wasser, Sirup und Methylcellulose. Ferner können die Formulierungen pharmazeutisch akzeptable Träger oder übliche Hilfsstoffe, wie Gleitmittel, beispielsweise Talg, Magnesiumstearat und Mineralöl; Netzmittel; emulgierende und suspendierende Mittel; konservierende Mittel, wie Methyl- und Propylhydroxybenzoate; Antioxidantien; Antireizstoffe; Chelatbildner; Dragierhilfsmittel; Emulsionsstabilisatoren Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle umfassen. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, dargestellt ist.

Für eine möglichst wirksame Applikation der erfindungsgemäßen Verbindungen werden Zusammensetzungen verschiedener Art bereitgestellt. Allen diesen Zusammensetzungen ist gemeinsam, daß die erfindungsgemäßen Verbindungen in Kombination mit einem organischen Träger stehen.

Eine bevorzugte Ausführungsform solcher Zusammensetzungen sieht die Assoziation der erfindungsgemäßen Verbindungen in Form von uni- bis oligolamellaren Liposomen mit einem Durchmesser von maximal 0.4 µm vor. Zur Liposomenbildung können alle an sich bekannten Verfahren der Liposomendarstellung verwendet werden, wie beispielsweise Ultraschall, Gelchromatographie, Detergenzanalyse, Hochdruckfiltration. Die jeweils eingeführten lipophilen Reste beeinflussen maßgeblich die Größe und Stabilität der Liposomen, die sich aus den jeweiligen Glycerylnucleotiden zusammen mit weiteren Lipidkomponenten bilden (vgl. auch Liposomes: From Physical Structure to Therapeutic Applications in: Research monographs in cell and tissue physiology Bd.7, G.G. Knight Hrsg., Elsevier (1981).

Eine weitere bevorzugte Möglichkeit, die erfindungsgemäßen Verbindungen mit einem organischen Träger zu verbinden, ist der Einschluß der Verbindungen in biologisch verträgliche Nanopartikel. Als Nanopartikel werden organisch-chemische Polymere bezeichnet, denen bei der Polymerisation die erfindungsgemäßen Verbindungen zugesetzt werden, so daß sie mit einer gewissen Effizienz in das Nanopartikel eingeschlossen werden (vgl. Bender et al., Antimicrobial agents and Chemotherapy (1996), 40 (6) 1467-1471).

Die Zusammensetzung wird in einer bevorzugten Ausführungsform mit Komponenten ausgeführt, die sich in den zu therapierenden Zellen und/oder Organen spezifisch anreichern. Dabei kann beispielsweise dre Zusammensetzung der Liposomen so gewählt werden, daß die Liposomen zusätzlich mit Molekülen wie z.B. Antikörper, geladene Lipide, mit hydrophilen Kopfgruppen modifizierte Lipide versehen werden, damit die Zusammensetzung sich bevorzugt in den zu therapierenden Zellen und/oder Organen anreichert. Eine solche Zusammensetzung mit spezifisch gegen Tumorzellen, virusinfizierten Zellen und/oder Organe gerichteten Molekülen erhöht die therapeutische Wirkung der Arzneimittel und reduziert gleichzeitig die Toxizität für nicht infizierte Gewebe.

Die Zusammensetzungen können zu einem Mittel verarbeitet werden, das neben den erfindungsgemäßen Verbindungen und gegebenenfalls dem organischen Träger weiterhin übliche Träger- und/oder Verdünnungsmittel und/oder Hilfsstoffe enthält. Übliche Trägermittel sind beispielsweise Mannit, Glucose, Dextrose, Albumine oder ähnliches, während als Verdünnungsmittel im wesentlichen physiologische Kochsalzlösungen oder eine 5%ige Glucoselösung dient. Weiterhin ist es üblich, die Lösungen mit geeigneten Reagenzien, beispielsweise Phosphaten, abzupuffern. Darüberhinaus können alle weiteren, für die Zubereitung von pharmazeutischen Mitteln üblichen Mittel hinzugesetzt werden, vorausgesetzt, sie greifen die Zusammensetzung aus dem organischen Träger und den erfindungsgemäßen Verbindungen nicht an. Das Mittel kann als Infusionslösung aber auch oral verabreicht werden.

Durch die Überführung in amphiphile Glycerylnucleotide lassen sich aber nicht nur die enzymatische Hydrolysebeständigkeit erhöhen und die Applikationsformen deutlich erweitern, sondern überraschenderweise auch zytosstatische und virusstatische Wirkungen optimieren.

Die amphiphilen Glycerylnucleotide lassen sich gegen maligne Erkrankungen der blutbildenden Zellen und solide Tumore einsetzen. Durch die verbesserte zytostatische Wirkung treten sehr viel weniger gravierende Nebenwirkungen auf. Es können höhere Dosen der zytostatisch wirksamen erfindungsgemäßen Verbindungen eingesetzt werden und es kann in zeitlichen Intervallen therapiert werden.

Überraschenderweise zeigen die erfindungsgemäßen Nucleosidphosphat-Analoga auch virustatische Wirkungen, so daß sie in der Chemotherapie von virusbedingten Infektionen und zur Überwindung von Arzneimittelresistenzen wie beispielsweise für Herpes, Hepatitis und AIDS verwendbar sind.

Ein weiterer Gegenstand der Erfindung betrifft daher die Verwendung erfindungsgemäßer Verbindungen zur Behandlung von Krebserkrankungen, wie Leukämie, Lungenkrebs, Darmkrebs, Krebs des Zentralnervensystems, Melanomen, Ovarialkrebs, Nierenkrebs, Prostatakrebs und Brustkrebs; sowie zur Behandlung viraler Erkrankungen, wie AIDS, Hepatitis A, B und C und Herpes .

Die folgenden Beispiele erläutern die Erfindung ohne diese jedoch zu beschränken.

### Beispiel 1

### Darstellung von 3'-Azido-2',3'-didesoxythymidylyl-(5'→2)-1-O-octadeyl-racglycerol-3-(hydroxycarbonyl)-phosphonat

### a) Herstellung des Ausgangsmaterials

2.5 g (3.8 mmol) 3-O-(4-Monomethoxytrityl)-1-O-octadecyl-rac-glycerol-2-hydrogenphosphonat werden zusammen mit 1 g (3.8 mmol) 3'-Azido-2',3'-didesoxythymidin in 15 ml wasserfreiem Pyridin gelöst. Die auf ca. 10 °C abgekühlte Lösung wird unter Feuchtigkeitsausschluß mit 2.3 ml (19 mmol) Pivaloylchlorid versetzt und 7 min. bei Raumtemperatur gerührt. Anschließend werden zu dem auf 0 °C gekühlten Reaktionsgemisch nacheinander 1 ml Wasser und 20 ml einer 0.2 M Lösung von lod in THF zugegeben, das anschließend ohne Kühlung 1 h gerührt wird. Überschüssiges lod wird durch Zusatz von festem Natriumhydrogensulfit reduziert, bevor der Reaktionsansatz im Rotationsverdampfer zu einem Sirup konzentriert wird, der in 80 ml Dichlormethan aufgenommen und dreimal gegen 50 ml Wasser/Methanol 1:1; V:V ausgeschüttelt wird. Die organische Phase wird im Rotationsverdampfer zu einem Sirup konzentriert, der noch dreimal mit Toluol coevaporiert wird. Zum Austauch der 4-Monomethoxytritylgruppe gegen Wasserstoff wird der Sirup in 50 ml Essigsäure/ Wasser; 4:1; V:V gelöst, 5 min. auf 40 °C erwärmt und anschließend im Rotationsverdampfer wieder zum Sirup konzentriert, der noch zweimal mit Toluol coevaporiert wird, dann in 25 ml Chloroform/ Methanol; 98:2; V:V gelöst und an einer Kieselgelsäule mit einem Chloroform/ Methanol-Gradienten fraktioniert wird. Die produktenthaltenden Fraktionen werden im Rotationsverdampfer in 2 g (3 mmol) eines Schaums überführt, der im Laufmittelsystem Chloroform/ Methanol; 7: 3; V:V einen R_{f}-Wert von 0.48 aufweist.

### b) Herstellung des Endprodukts

Der gemäß a) erhaltene Schaum wird unter Rühren portionsweise zu einer auf 0 °C gekühlten Lösung von 1 g Ethoxycarbonylphosphonsäuredichlorid gelöst in 12 ml Phosphorsäuretrimethylester zugegeben. Die Reaktionsmischung wird weitere 4 h bei 0 °C gerührt, anschließend unter Kühlung mit 50 ml Ether und 50 ml Wasser versetzt und gut durchgeschüttelt. Die wässerige Phase wird abgetrennt, und die organische Phase wird nochmals mit Wasser ausgeschüttelt. Die vereinigten wässerigen Phasen werden unter Eiskühlung mit 2 M Natronlauge neutralisiert, zu einem Sirup konzentriert, der in Ether aufgenommen und unter Ultraschall kristallisiert. Der erhaltene Niederschlag wird abzentrifugiert, getrocknet, in 10 ml Wasser gelöst, mit 10 ml 2 N Natronlauge versetzt und 1 h bei Raumtemperatur gerührt. Die Reaktionslösung wird mit 2 N Salzsäure neutralisiert, im Vakuum konzentriert und an einer Sephadex-G₁₀-Säule entsalzt. Die produktenthaltenden Fraktionen werden lyophilisiert und ergeben 2.1 g 3'-Azido-2',3'-didesoxythymidylyl-(5'→2)-1-O-octadeyl-rac-glycerol-3-(hydroxycarbonyl)-hydrogenphosphonat in Form eines farblosen Pulvers, das auf der Kieselgeldünnschichtplatte, im Laufmittelsystem Chloroform/ Methanol; 6:4; V:V einen R_{f}-Wert von 0.1 aufweist. Die berechnete Molekülmasse (769.77) wird im lon-Spray-Massenspektrum durch den Molekülpeak (768.5) bestätigt.

### Beispiel 2

### Darstellung von Arabinocytidylyl-(5'→1)-2-O-octadecyl-rac-glycerylyl-(3→5')-5-fluoro-2'-desoxyuridin

### a) Herstellung des Ausgangsmaterials

4.8 g (7 mmol) 1-O-(4-Monomethoxytrityl)-2-O-octadecyl-rac-glycerol-3-hydrogenphosphonat werden zusammen mit 2 g (7 mmol) 3'-O-Acetyl-5-fluoro-2'desoxyuridin in 50 ml wasserfreiem Pyridin unter Zusatz von 4.3 ml Pivaloylchlorid in Analogie zu a) aus Beispiel 1 kondensiert und mit 32 ml einer 0.2 M Lösung von lod in THF oxidiert. Der nach der Kondensation erhaltene Sirup wird zum Austausch der 4-Monomethoxytritylgruppe gegen Wasserstoff in 70 ml Essigsäure/ Wasser; 4:1; V:V gelöst und 30 min. unter Rückfluß erhitzt. Die erkaltete Lösung wird im Rotationsverdampfer zum Sirup konzentriert, der noch zweimal mit Toluol coevaporiert, anschließend in 35 ml Dichlormethan/ Methanol; 9:1; V:V aufgenommen und an einer Kieselgelsäule mit einem Dichlormethan/ Methanol-Gradienten fraktioniert wird. Die produktenthaltenden Fraktionen werden im Vakuum in 3.0 g Schaum überführt, der auf der Kieselgeldünnschichtplatte im Laufmittelsystem Chloroform/ Methanol; 7:3; V:V einen R_{f}-Wert von 0.32 aufweist.

### b) Herstellung des Endprodukts

Der gemäß a) erhaltene Schaum und 2.8 g (4.3 mmol) 2',3'-Di-O-acetyl-N⁴-stearoylarabinocytidin-5'-hydrogenphosphonat werden in 35 ml wasserfreiem Pyridin unter Zusatz von 3 ml Pivaloylchlorid in Analogie zu a) aus Beispiel 1 kondensiert. Nach Zugabe von 2 ml Wasser wird durch Zusatz von 20 ml einer 0.2 M Lösung von lod in THF oxidiert. Das Reaktionsgemisch wird nach der Konzentration im Rotationsverdampfer in Dichlormethan aufgenommen und dreimal mit einer Lösung aus gesättigtem wässerigen NaCl/ Wasser/ Methanol; 1:1:2; V:V:V ausgeschüttelt. Die organische Phase wird im Vakuum zu einem Sirup konzentriert, der noch dreimal mit Toluol coevaporiert wird. Anschließend wird der Sirup in Dichlormethan/Methanol; 9:1; V:V aufgenommen und an einer Kieselgelsäule mit einem Dichlormethan/ Methanol-Gradienten fraktioniert. Die produktenthaltenden Fraktionen werden im Vakuum zu einem Sirup konzentriert, der in heißem Ethylacetat gelöst, bei 0°C kristallisiert. Der Niederschlag wird zum Austauch der Acylschutzgruppen gegen Wasserstoff, in wenig Dichlormethan/ Methanol; 1:1; V:V gelöst. Die Lösung wird mit Methanol, der bei Raumtemperatur mit Ammoniak gesättigt wurde, versetzt, 36 h verschlossen bei Raumtemperatur stehen gelassen und dann im Rotationsverdampfer bis zur eintretenden Kristallisation konzentriert. Das nach 12 h Stehen bei 0°C ausgefallende Arabinocytidylyl-(5'→1)-2-Ooctadecyl-rac-glycerylyl-(3→5')-5-fluoro-2'-desoxyuridin wird abgesaugt, mit Ether gewaschen und ergibt nach dem Trocknen 2.3 g eines weißen Pulvers, das auf der Kieselgeldünnschichtplatte im Laufmittelsystem Chloroform/ Methanol/ NH₃ 10:10:3; V:V:V einen R_{f}-Wert von 0.10 aufweist. Die berechnete Molekülmasse (957.93) wird im lon-Spray-Massenspektrum durch den Molekülpeak (957.0) bestätigt.

### Beispiel 3

### Darstellung von N⁴-Palmitoyl-3'-thia-2',3'-didesoxycytidylyl-(5'→1)-2-Ooctadecyl-rac-glycerylyl-(3→5')-3'-azido-2',3'-didesoxythymidin

### a) Herstellung des Ausgangsmaterials:

2.4 g (5.3 mmol) 1-O-Acetyl-2-O-octadecyl-rac-glycerol-3-hydrogenphosphonat und 1.4 g (5.3 mmol) 3'-Azido-2',3'-didesoxythymidin werden in 30 ml wasserfreiem Pyridin unter Zusatz von 3.5 ml (28.4 mmol) Pivaloylchlorid in Analogie zu a) aus Beispiel 1 kondensiert. Nach Zugabe von 2 ml Wasser wird mit von 25 ml einer 0.2 M Lösung von lod in THF oxidiert. Das Reaktionsgemisch wird im Rotationsverdampfer konzentriert, in 85 ml Chloroform aufgenommen und dreimal mit je 40 ml einer Lösung aus gesättigtem wässerigen NaCl/ Wasser/ Methanol; 1:1:2; V:V:V ausgeschüttelt. Die organische Phase wird dann zum Sirup konzentriert, der zum Tausch der Acetylgruppe gegen Wasserstoff 12 h mit Methanol behandelt wird, der bei Raumtemperatur mit Ammoniak gesättigt wurde. Nach der anschließenden Konzentration im Vakuum wird der Sirup in Dichlormethan aufgenommen und an einer Kieselgelsäule mit einem Dichlormethan/ Methanl-Gradienten fraktioniert. Die produktenthaltenden Fraktionen werden im Vakuum zum Sirup konzentriert, der in Ethanol gelöst, bei -25 °C 3.0 g farblose Kristalle bildet, die im Laufmittelsystem Chlorform/ Methanol; 7:3; V:V; einen R_{f}-Wert von 0.26 aufweisen.

### b) Herstellung des Endprodukts

Die gemäß a) erhaltenen Kristalle und 2.4 g (4.5 mmol) N⁴-Palmitoyl-3'-thia-2',3'didesoxycytidin-5'-hydrogenphosphonat werden in 40 ml trockenem Pyridin unter Zusatz von 3.0 ml Pivaloylchlorid in Analogie zu a) aus Beispiel 1 kondensiert und mit 20 ml einer 0.2 M Lösung von lod in THF oxidiert. Der hierbei erhaltene Sirup wird in 100 ml Chloroform/ Methanol; 9:1; V:V gelöst und an einer Kieselgelsäule mit einem Chloroform/ Methanol-Gradienten fraktioniert. Die produktenthaltenden Fraktionen werden im Vakuum zu einem Sirup konzentriert, aus dem nach Methanolzugabe 2.5 g N⁴-Palmitoyl-3'-thia-2',3'-didesoxycytidylyl-(5'→1)-2-Ooctadecyl-rac-glycerylyl-(3→5')-3'-azido-2',3'-didesoxythymidin in Form eines weißen Pulvers auskristallisieren, das auf der Kieselgeldünnschichtplatte, im Laufmittelsystem Chloroform/ Methanol; 7:3; V:V einen R_{f}-Wert von 0.20 aufweist. Die berechnete Molekülmasse (1203.5) wird im lon-Spray-Massenspektrum durch den Molekülpeak (1202.5) bestätigt.

### Beispiel 4

Darstellung von 3'-Azido-2',3'-didesoxythymidylyl-(5'→ 1)-2-O-octadecyl-racglycerylyl-(3→5')-N⁴-palmitoyl-2',3'-didesoxycytidin Herstellung des Endprodukts

Die gemäß a) aus Beispiel 3 erhaltenen Kristalle und 2.3 g (4.5 mmol) N⁴-Palmitoyl-2',3'-didesoxycytidin-5'-hydrogenphosphonat werden in 40 ml wasserfreiem Pyridin unter Zusatz von 3.0 ml (24.4 mmol) Pivaloylchlorid in Analogie zu a) aus Beispiel 1 kondensiert und mit 20 ml einer 0.2 M Lösung von lod in THF oxidiert. Der nach der Kondensation erhaltene Sirup wird in 130 ml heißem Ethanol gelöst, filtriert und bei -25 °C kristallisieren 5.1 g 3'-Azido-2',3'-didesoxythymidylyl-(5'→1)-2-Ooctadecyl-rac-glycerylyl-(3→5')-N⁴-palmitoyl-2',3'-didesoxycytidin in Form farbloser Kristalle aus, die auf der Kieselgelplatte im Laufmittelsystem Chloroform/ Methanol; 6:4; V:V; einen R_{f}-Wert von 0.26 aufweisen. Die berechnete Molekülmasse (1185.40) wird im lon-Spray-Massenspektrum durch den Molekülpeak (1184.0) bestätigt.

### Beispiel 5

### Darstellung von N⁴-Palmitoyl-2',3'-didesoxycytidylyl-(5'→1)-2-O-palmitoylrac-glycerylyl-(3→ 5')-3'-azido-2',3'-didesoxythymidin

### a) Herstellung des Ausgangsmaterials

3.2 g (7.2 mmol) 1-O-Acetyl-2-O-palmitoyl-rac-glycerol-3-hydrogenphosphonat und 2.0 g (7.2 mmol) 3'-Azido-2',3'-didesoxythymidin werden in 60 ml trockenem Pyridin unter Zusatz von 4.5 ml (36.6 mmol) Pivaloylchlorid in Analogie zu a) aus Beispiel 1 kondensiert und mit 32 ml einer 0.2 M Lösung von lod in THF oxidiert. Der nach der Kondensation erhaltene Sirup wird in 75 ml Chloroform/ Methanol; 9:1; V:V aufgenommen und an einer Kieselgelsäule mit einem Chloroform/Methanol-Gradienten fraktioniert. Die produktenthaltenden Fraktionen werden im Vakuum konzentriert, in 15 ml Chloroform aufgenommen, mit 120 ml Methanol, der bei Raumtemperatur mit Ammoniak gesättigt wurde versetzt und 90 min. bei Raumtemperatur stehen gelassen. Anschließend wird der Reaktionsansatz im Vakuum konzentriert und mit Toluol zur Trockne coevaporiert. Der Rückstand wird in Chloroform/ Methanol; 9:1; V:V aufgenommen und an einer Kieselgelsäule mit einem Chloroform/ Methanol-Gradienten fraktioniert. Die produktenthaltenden Fraktionen werden im Vakuum zu 2.7 g eines Schaums konzentriert, der im Laufmittelsystem Chlorform/ Methanol; 7:3; V:V einen R,-Wert von 0.24 aufweist.

### b) Herstellung des Endprodukts

Der gemäß a) erhaltene Schaum und 2.1 g (4.1 mmol) N⁴-Palmitoyl-2',3'didesoxycytidin-5'-hydrogenphosphonat werden in 35 ml trockenem Pyridin unter Zusatz von 25 ml (20.3 mmol) Pivaloylchlorid in Analogie zu a) aus Beispiel 1 kondensiert und mit 20 ml einer 0.2 M Lösung von lod in THF oxidiert. Der nach der Kondensation erhaltene Sirup wird in 50 ml Chloroform/ Methanol; 95:5; V:V aufgenommen und an einer Kieselgelsäule mit einem Chloroform/ Methanol-Gradienten fraktioniert. Die produktenthaltenden Fraktionen werden im Vakuum zu einem Sirup konzentriert, aus dem bei Methanolzugabe 2.3 g N⁴-Palmitoyl-2',3'didesoxycytidylyl-(5'→1 )-2-O-palmitoyl-rac-glycerylyl-(3→5')-3'-azido-2',3'-didesoxythymidin in Form eines weißen Pulvers auskristallisieren, das auf der Kieselgeldünnschichtplatte, im Laufmittelsystem Chloroform/ Methanol; 6:4; V:V einen R_{f}-Wert von 0.25 aufweist. Die berechnete Molekülmasse (1171.32) wird im lon-Spray-Massenspektrum durch den Molekülpeak (1170.0) bestätigt

### Beispiel 6

### Darstellung von 3'-Azido-2',3'-didesoxythymidylyl-(5'→1)-2-O-octadecyl-racglycerylyl-(3→5')-2',3'-didesoxyinosin

### a) Herstellung des Ausgangsmaterials

Das gemäß a) aus Beispiel 3 erhaltene Ausgangsmaterial wird in einer Mischung aus 10 ml trockenem Pyridin und 40 ml trockenem Dioxan gelöst, mit 1.1 g (5.3 mmol) Salicylchlorophosphit versetzt und 1 h bei Raumtemperatur geschüttelt. Anschließend wird die Reaktionsmischung auf 0 °C gekühlt, mit 2 ml Wasser versetzt, weitere 15 min. bei Raumtemperatur geschüttelt und im Vakuum zu einem Sirup konzentriert, der in 120 ml Wasser aufgenommen und dreimal mit je 40 ml Ethylacetat ausgeschüttelt wird. Die wässerige Phase wird im Vakuum konzentriert und lyophilisiert. Es werden 2.5 g eines weißen Pulvers erhalten, das auf der Kieselgelplatte, im Laufmittelsystem Chloroform/ Methanol; 7:3; V:V einen R_{f}-Wert von 0.15 aufweist.

### b) Herstellung des Endprodukts

Das gemäß a) erhaltene Lyophilisat wird mit 0.8 g (3.4 mmol) 2',3'-Didesoxyinosin in 25 ml Pyridin unter Zusatz von 2.0 ml (16.3 mmol) Pivaloylchlorid in Analogie zu a) aus Beispiel 1 kondensiert und mit 16 ml einer 0.2 M Lösung von lod in THF oxidiert. Der nach der Kondensation erhaltene Sirup wird in 50 ml Chloroform/Methanol; 9:1; V:V aufgenommen und an einer Kieselgelsäule mit einem Chloroform/ Methanol-Gradienten fraktioniert. Die produktenthaltenden Fraktionen werden im Vakuum zu einem Sirup konzentriert aus dem bei Etherzugabe 1.1 g 3'-Azido-2',3'-didesoxythymidylyl-(5'→1)-2-O-octadecyl-rac-glycerylyl-(3→5')-2',3'didesoxyinosin in Form weißer Kristalle auskristallisieren, die auf der Kieselgelplatte, im Laufmittelsystem Chloroform/ Methanol; 1:1; V:V einen R_{f}-Wert von 0.21 aufweisen. Die berechnete Molekülmasse (971.99) wird im lon-Spray-Massenspektrum durch den Molekülpeak (970.5) bestätigt.

### Beispiel 7

### Darstellung von 3'-Fluoro-2',3'-didesoxythymidylyl-(5'→1)-2-O-octadecyl-racglycerylyl-(3→5')-3'-azido-2',3'-didesoxythymidin

### a) Herstellung des Ausgangsmaterials

4,3 g (9,5 mmol) 1-O-Acetyl-2-O-octadecyl-rac-glycerol-3-hydrogenphosphonat werden zusammen mit 2,3 g (9.5 mmol) 3'-Fluoro-2',3'-didesoxythymidin in 25 ml Pyridin unter Zusatz von 5,7 ml Pivaloylchlorid in Analogie zu a) aus Beispiel 1 kondensiert und mit 45 ml einer 0.2 M Lösung von lod in THF oxidiert. Der nach der Kondensation erhaltene Sirup wird in wenig Chloroform aufgenommen mit 200 ml Methanol, der bei Raumtemperatur gesättigt wurde, versetzt, kräftig geschüttelt und dann über Nacht bei Raumtemperatur belassen. Das Reaktionsgemisch wird anschließend im Vakuum bis zum Eintritt der Kristallisation konzentriert. Der nach 12 h Stehen bei -25 °C erhaltene Niederschlag wird abgesaugt und getrocknet. Es werden 3.4 g eines weißen Pulvers erhalten, das auf der Kieselgeldünnschichtplatte, im Laufmittelsystem Chloroform/ Methanol; 6:4; V:V einen R_{f}-Wert von 0,71 aufweist.

### b) Herstellung des Endprodukts

3.4 g (5.2 mmol) des gemäß a) erhaltenen Pulvers und 1.7 g (2.3 mmol) 3'-Azido-2',3'-didesoxythymidin-5'-hydrogenphosphonat werden in 45 ml trockenem Pyridin unter Zusatz von 3.5 ml Pivaloylchlorid in Analogie a) aus Beispiel 1 kondensiert und mit 25 ml einer 0.2 M Lösung von lod in THF oxidiert. Der nach der Kondensation erhaltene Sirup wird in 100 ml Chloroform/ Methanol; 9:1; V:V gelöst und an einer Kieselgelsäule mit einem Chloroform/ Methanol-Gradienten fraktioniert. Die produktenthaltenden Fraktionen werden im Vakuum zu einem Sirup konzentriert, aus dem bei Methanolzugabe 2.7 g 3'-Fluoro-2',3'-didesoxythymidylyl-(5'→1)-2-Ooctadecyl-rac-glycerylyl-(3→5')-3'-azido-2',3'-didesoxythymidin in Form weißer Kristalle erhalten werden, die auf der Kieselgeldünnschichtplatte, im Laufmittelsystem Chloroform/ Methanol; 6:4; V:V einen R_{f}-Wert von 0.45 aufweisen. Die berechnete Molekülmasse (979.91) wird im lon-Spray-Massenspektrum durch den Molekülpeak (979.0) bestätigt.

### Beispiel 8

Herstellung einer Zusammensetzung, enthaltend einen organischen Träger, eines der erfindungsgemäßen amphiphilen Glycerylnucleotide und Cholesterol.

### 1. Zusammensetzung der Liposomen

### Liposomen mit 10 mg der erfindungsgemäßen Verbindung pro ml.

1 ml Liposomen enthalten: 100 mg Phosphaditylcholin, 10 mg Chlolesterol und 10 mg der erfindungsgemäßen Verbindung dispergiert in 0.9 % Kochsalzlösung, die, wenn nötig, zum Beispiel mit Phosphatpuffer auf einen gewünschten pH-Wert eingestellt ist. Anstelle der gegenbenenfalls gepufferten Kochsalzlösung kann auch ein 67 nM physiologischer Phosphatpuffer oder eine 5%ige Glucoselösung verwendet werden.

### 2. Herstellung der Liposomenpräparate durch Ultraschall

Herstellung von 2.5 ml Liposomendispersion, die 10 mg der erfindungsgemäßen Verbindung pro ml enthalten. 1n einem 100 ml Rundkolben werden 250 mg Soja-Phophaditylcholin in 5 ml t-Butanol gelöst und mit 25 mg Cholesterol versetzt, das sich unter Erwärmung (50 °C) löst. Zu dieser Lösung werden 25 mg der erfindungsgemäßen Verbindung, die in der geeigneten Menge DMSO (0.25 ml - 1 ml) gelöst sind, zugesetzt, und die resultierende Lösung wird im Rotationsverdampfer auf die Hälfte konzentriert und anschließend lyophilisiert. Das erhaltene Lyophilisat wird mit 2.5 ml Wasser versetzt und durch Ultraschall in eine Suspension überführt, die anschließend nochmals lyophilisiert wird. Das Lyophilisat wird in 2.5 ml einer 0.9 %igen NaCl-Lösung suspendiert und unter Kühlung in einem dickwandigen Zentrifugenglas 1 h mit der Ultraschallspitze (3 mm) eines Branson Sonifiers 250 beschallt. Man erhält eine opaleszente Liposomendispersion mit der Zusammensetzung 100 mg SPC, 10 mg Cholesterol und 10 mg der erfindungsgemäßen Verbindung pro ml.

### Beispiel 9

### In vitro Untersuchung des Anti-HIV-Effekts einer erfindungsgemäßen Verbindung

Die *in vitro* virusstatische (anti-HIV) Wirkung der erfindungsgemäßen Verbindung N⁴-Palmitoyl-2',3'-didesoxycytidylyl-(5'→1)-2-O-palmitoyl-rac-glycerylyl-(3→5')-3'azido-2',3'-didesoxythymidin (NSC 704532-F/1) läßt sich in folgender Versuchsanordnung zeigen. Als Testsystem dienen T4-Lymphozyten (Zellinie: CEM-SS), die durch HIV innerhalb von zwei Replikationszyklen getötet werden. Detektiert wird die Hemmung des Zelltods. Die erfindungsgemäße Verbindung wird in DMSO gelöst und diese Lösung wird mit Zellkulturmedium im Verhältnis 1: 100 verdünnt. Anschließend werden halblogarithmische (log₁₀) Verdünnungen hergestellt. Zu diesen Lösungen werden T4-Lymphozyten und nach einem bestimmten Zeitraum HI-Viren zugesetzt. Man erhält eine Verdünnung von mindestens 1:200 bezogen auf die ursprüngliche Lösung der erfindungsgemäßen Verbindung in DMSO. Als Toxizitätskontrolle dienen nicht infizierte T4-Lymphozyten, die nur mit der erfindungsgemäßen Verbindung inkubiert werden. Als weitere Standard-Kontrollen dienen infizierte und nicht infizierte T4-Zellen ohne erfindungsgemäße Verbindung. Die Zellkulturen werden 6 Tage lang bei 37 °C in einer 5 %-igen CO₂-Atmosphäre inkubiert, dann wird Tetrazoliumsalz (XTT) zu allen Zellkulturen zugesetzt und die Formazan-Farbreaktion tritt bei lebensfähigen Zellen auf. Alle Zellkulturen werden spekrophotometrisch untersucht um die Formazan Produktion zu quantifizieren. Darüber hinaus werden einzelne Kulturen mikroskopisch untersucht um die Anti-HIV Aktivität zu bestätigen. Die virusinfizierten Zellen, die mit der erfindungsgemäßen Verbindung behandelt wurden, werden mit den Standard auf derselben Platte verglichen. Die im Rahmen eines National Cancer Institute Developmental Therapeutics Program ermittelten in vitro Anti-HIV-Aktivitätsdaten sind in folgender Tabelle zusammengefaßt. Aus den Daten folgt, daß das Zellwachstum zu 50 % (IC₅₀) bei > 1.00x10⁻⁶ Mol der erfindungsgemäßen Verbindung, der 50 % Infektionsschutz (EC₅₀) bei 1.79x10⁻⁸ Mol und die therapeutische Breite TI₅₀ (IC/EC) bei > 5.59x10⁺¹ liegt.

**Tabelle**

| Testergebnisse der anti-HIV Aktivität von N⁴-Palmitoyl-2',3'-didesoxycytidylyl-(5'→1)-2-O-palmitoyl-rac-glycerylyl-(3→5')-3'-azido-2',3'-didesoxythymidin | | | |
|---|---|---|---|
| Dosis (molar) | Prozentualer Schutz | Prozent der Kontrolle | |
| | | Infiziert | nichtinfiziert |
| 3,17 * 10⁻¹⁰ | 1,88 | 5,80 | 105,43 |
| 1,00 * 10⁻⁹ | 0,22 | 4,21 | 105,03 |
| 3,17 * 10⁻⁹ | 10,68 | 14,25 | 101,22 |
| 1,00 * 10⁻⁸ | 20,44 | 23,62 | 99,64 |
| 3,16 * 10⁻⁸ | 79,40 | 80,22 | 104,96 |
| 1,00 * 10⁻⁷ | 106,32 | 106,07 | 112,26 |
| 3,16 * 10⁻⁷ | 104,33 | 104,16 | 112,02 |
| 1,00 * 10⁻⁶ | 114,05 | 113,49 | 119,96 |

### Beispiel 10

### In vitro zytostatische Wirkung erfindungsgemäßer Verbindungen.

### a) Versuchsanordnung

Als Testsystem dienen Tumorzellinien, deren 50%ige (GI50), beziehungsweise 100%ige (TGI) Wachstumshemmung durch die erfindungsgemäßen Verbindung bei verschiedenen Konzentrationen ermittelt wird. Ferner wird die Toxizität (LC₅₀) der Verbindung auf diese Zellen ermittelt. Eine Serie von Mikrotiterplatten wird am Tag 0 mit den Tumorzellen beimpft und für 24 h vorinkubiert. Danach wird die erfindungsgemäße Verbindung den Zellen in fünf jeweils 10-fach verdünnten Konzentrationen zugegeben, ausgehend von der höchsten löslichen Konzentration. Es folgt eine 48-stündige Inkubation, an deren Ende die Zellen in situ fixiert, gewaschen und getrocknet werden. Anschließend wird Sulforhodamine B (SRB), ein pinker Farbstoff, der an die fixierten Zellen gebunden wird, zugefügt und die Zellen erneut gewaschen. Der verbleibende Farbstoff spiegelt die adhärente Zellmasse wieder und wird spektrometrisch bestimmt. Die automatisch erfaßten Daten werden per Computer ausgewertet, und führen zu folgenden Ergebnissen:

### b) Ergebnisse

### b1) Für die erfindungsgemäße amphiphile Verbindung Arabinocytidylyl-(5'→1)-2-O-octadecyl-rac-glycerylyl-(3→5')-5-fluoro-2'-desoxyuridin (NSC 698688-A/1)

| In-Vitro Testergebnisse von Arabinocytidylyl-(5'→1)-2-O-octadecyl-rac-glycerylyl-(3→5')-5-fluoro-2'-desoxyuridin (NSC 698688-A/1) | | | |
|---|---|---|---|
| Zellinie | GI₅₀ in mol/l | TGI in mol/l | LC₅₀ in mol/l |
| Leukämie | | | |
| CCRF-CEM | 1.28E-06 | > 1.00E-04 | > 1.00E-04 |
| HL-60(TB) | 3.66E-06 | > 1.00E-04 | > 1.00E-04 |
| K-562 | 3.35E-06 | > 1.00E-04 | > 1.00E-04 |
| MOLT-4 | 4.67E-07 | > 1.00E-04 | > 1.00E-04 |
| RPMI-8226 | 1.65E-06 | > 1.00E-04 | > 1.00E-04 |
| SR | | > 1.00E-04 | > 1.00E-04 |

| Lungenkrebs | | | |
|---|---|---|---|
| A549/ATCC | 3.46E-07 | > 1.00E-04 | > 1.00E-04 |
| EKVX | > 1.00E-04 | > 1.00E-04 | > 1.00E-04 |
| HOP-62 | 6.99E-07 | > 1.00E-04 | > 1.00E-04 |
| HOP-92 | 8.02E-08 | 8.99E-05 | > 1.00E-04 |
| NCl-H226 | 7.59E-05 | > 1.00E-04 | > 1.00E-04 |
| NCI-H23 | 7.23E-06 | > 1.00E-04 | > 1.00E-04 |
| NCI-H322M | 3.56E-05 | > 1.00E-04 | > 1.00E-04 |
| NCI-H460 | 4.66E-08 | 2.79E-05 | > 1.00E-04 |
| NCI-H522 | 1.62E-05 | > 1.00E-04 | > 1.00E-04 |

| Darmkrebs | | | |
|---|---|---|---|
| COLO205 | 1.05E-05 | 3.88E-05 | > 1.00E-04 |
| HCC-2998 | 5.88E-08 | 3.50E-05 | > 1.00E-04 |
| HCT-116 | 4.90E-06 | > 1.00E-04 | > 1.00E-04 |
| HCT-15 | 1.44E-05 | > 1.00E-04 | > 1.00E-04 |
| HT29 | 1.25E-05 | > 1.00E-04 | > 1.00E-04 |
| KM12 | 7.47E-05 | > 1.00E-04 | > 1.00E-04 |
| SW-620 | 6.28E-05 | > 1.00E-04 | > 1.00E-04 |

| Krebs des Zentralen Nervensystems | | | |
|---|---|---|---|
| SF-268 | 7.45E-07 | > 1.00E-04 | > 1.00E-04 |
| SF-295 | 7.99E-07 | > 1.00E-04 | > 1.00E-04 |
| SF-539 | 4.88E-07 | 1.03E-05 | 8.03E-05 |
| SNB-19 | 9.19E-07 | 2.37E-05 | 6.41E-05 |
| SNB-75 | 2.04E-05 | > 1.00E-04 | > 1.00E-04 |
| U251 | 3.14E-06 | 2.48E-05 | 7.38E-05 |

| Melanom | | | |
|---|---|---|---|
| LOX IMVI | 1.40E-06 | > 1.00E-04 | > 1.00E-04 |
| MALME-3M | > 1.00E-04 | > 1.00E-04 | > 1.00E-04 |
| M14 | 3.53E-06 | 8.62E-05 | > 1.00E-04 |
| SK-MEL-2 | > 1.00E-04 | > 1.00E-04 | > 1.00E-04 |
| SK-MEL-28 | 5.78E-05 | > 1.00E-04 | > 1.00E-04 |
| SK-MEL-5 | > 1.00E-04 | > 1.00E-04 | > 1.00E-04 |
| UACC-257 | 4.98E-05 | > 1.00E-04 | > 1.00E-04 |
| UACC-62 | 1.77E-06 | > 1.00E-04 | > 1.00E-04 |

| Eierstockkrebs | | | |
|---|---|---|---|
| IGROV1 | 1.36E-05 | > 1.00E-04 | > 1.00E-04 |
| OVCAR-3 | > 1.00E-04 | > 1.00E-04 | > 1.00E-04 |
| OVCAR-4 | 5.85E-05 | > 1.00E-04 | > 1.00E-04 |
| OVCAR-5 | 8.96E-05 | > 1.00E-04 | > 1.00E-04 |
| OVCAR-8 | 9.68E-07 | > 1.00E-04 | > 1.00E-04 |
| SK-OV-3 | 3.63E-05 | > 1.00E-04 | > 1.00E-04 |

| Nierenkrebs | | | |
|---|---|---|---|
| 768-0 | 8.54E-07 | 1.34E-05 | 4.44E-05 |
| A498 | 3.19E-07 | > 1.00E-04 | > 1.00E-04 |
| ACHN | 4.66E-07 | > 1.00E-04 | > 1.00E-04 |
| CAKI-1 | 1.04E-05 | > 1.00E-04 | > 1.00E-04 |
| RXF 393 | 1.02E-05 | 2.86E-05 | 8.00E-05 |
| SN12C | 1.33E-05 | > 1.00E-04 | > 1.00E-04 |
| TK-10 | 2.29E-05 | > 1.00E-04 | > 1.00E-04 |
| UO-31 | 2.13E-05 | > 1.00E-04 | > 1.00E-04 |

| Prostatakrebs | | | |
|---|---|---|---|
| PC-3 | 1.15E-05 | 8.64E-05 | > 1.00E-04 |
| DU-145 | 2.68E-06 | > 1.00E-04 | > 1.00E-04 |

| Brustkrebs | | | |
|---|---|---|---|
| MCF7 | 1.70E-07 | > 1.00E-04 | > 1.00E-04 |
| NCI/ADR-RES | · | > 1.00E-04 | > 1.00E-04 |
| MDA-MB-231/ATCC | > 1.00E-04 | > 1.00E-04 | > 1.00E-04 |
| HS 578T | > 1.00E-04 | > 1.00E-04 | > 1.00E-04 |
| MDA-MB-435 | 3.38E-05 | > 1.00E-04 | > 1.00E-04 |
| MDA-N | > 1.00E-04 | > 1.00E-04 | > 1.00E-04 |
| BT-549 | 1.45E-05 | 8.69E-05 | > 1.00E-04 |
| T-47D | > 1.00E-04 | > 1.00E-04 | > 1.00E-04 |

Aufgrund obiger in vitro Testergebnisse eignet sich dieser Wirkstoff bevorzugt zur Therapie von Tumoren der Lunge, des Darms, des Zentralnervensystems und der Niere.

### b2) Für die erfindungsgemäße nicht-amphiphile Verbindung 2',3'-Didesoxycytidylyl-(5'→1)-rac-glycerylyl-(3→5')-5-fluoro-2'-desoxyuridin (NSC 704533):

| In-Vitro Testergebnisse von 2',3'-Didesoxycytidylyl-(5'→1)-*rac*-glycerylyl-(3→5')-5-fluoro-2'-desoxyuridin (NSC 704533) | | | |
|---|---|---|---|
| Zellinie | GI₅₀ in mol/l | TGI in mol/l | LC₅₀ in mol/l |
| Leukämie | | | |
| CCRF-CEM | . | > 1.00E-04 | > 1.00E-04 |
| HL-60 (TB) | 7.15E-08 | > 1.00E-04 | > 1.00E-04 |
| K-562 | 2.57E-06 | > 1.00E-04 | > 1.00E-04 |
| MOLT-4 | 1.04E-07 | > 1.00E-04 | > 1.00E-04 |
| RPMI-8226 | 1.10E-05 | > 1.00E-04 | > 1.00E-04 |
| SR | < 1.00E-08 | > 1.00E-04 | > 1.00E-04 |

| Lungenkrebs | | | |
|---|---|---|---|
| A549/ATCC | 4.77E-08 | > 1.00E-04 | > 1.00E-04 |
| EKVX | > 1.00E-04 | > 1.00E-04 | > 1.00E-04 |
| HOP-62 | 9.02E-07 | > 1.00E-04 | > 1.00E-04 |
| NCI-H226 | > 1.00E-04 | > 1.00E-04 | > 1.00E-04 |
| NCl-H23 | 9.79E-08 | > 1.00E-04 | > 1.00E-04 |
| NCI-H322M | 1.95E-06 | > 1.00E-04 | > 1.00E-04 |
| NCI-H460 | < 1.00E-08 | > 1.00E-04 | > 1.00E-04 |
| NCI-H522 | 7.46E-07 | > 1.00E-04 | > 1.00E-04 |

| Darmkrebs | | | |
|---|---|---|---|
| COLO 205 | 1.77E-05 | > 1.00E-04 | > 1.00E-04 |
| HCT-116 | 3.74E-07 | > 1.00E-04 | > 1.00E-04 |
| HCT-15 | 1.79E-05 | > 1.00E-04 | > 1.00E-04 |
| HT29 | 1.88E-06 | > 1.00E-04 | > 1.00E-04 |
| KM12 | 1.45E-05 | > 1.00E-04 | > 1.00E-04 |
| SW-620 | > 1.00E-04 | > 1.00E-04 | > 1.00E-04 |

| Krebs des Zentralen Nervensystems | | | |
|---|---|---|---|
| SF-268 | < 1.00E-08 | > 1.00E-04 | > 1.00E-04 |
| SF-295 | 3.45E-08 | > 1.00E-04 | > 1.00E-04 |
| SF-539 | 2.16E-08 | > 1.00E-04 | > 1.00E-04 |
| SNB-19 | 2.50E-07 | > 1.00E-04 | > 1.00E-04 |
| SNB-75 | 1.59E-06 | > 1.00E-04 | > 1.00E-04 |
| U251 | 1.26E-06 | > 1.00E-04 | > 1.00E-04 |

| Melanom | | | |
|---|---|---|---|
| LOX IMVI | 1.01E-08 | > 1.00E-04 | > 1.00E-04 |
| MALME-3M | > 1.00E-04 | > 1.00E-04 | > 1.00E-04 |
| M14 | 2.03E-07 | > 1.00E-04 | > 1.00E-04 |
| SK-MEL-2 | 7.94E-05 | > 1.00E-04 | > 1.00E-04 |
| SK-MEL-28 | 3.79E-05 | > 1.00E-04 | > 1.00E-04 |
| SK-MEL-5 | 6.89E-07 | > 1.00E-04 | > 1.00E-04 |
| UACC-257 | > 1.00E-04 | > 1.00E-04 | > 1.00E-04 |
| UACC-62 | 5.50E-07 | > 1.00E-04 | > 1.00E-04 |

| Eierstockkrebs | | | |
|---|---|---|---|
| IGROV1 | 6.80E-05 | > 1.00E-04 | > 1.00E-04 |
| OVCAR-3 | 6.54E-05 | > 1.00E-04 | > 1.00E-04 |
| OVCAR-4 | > 1.00E-04 | > 1.00E-04 | > 1.00E-04 |
| OVCAR-5 | 1.26E-05 | > 1.00E-04 | > 1.00E-04 |
| OVCAR-8 | 1.41E-07 | > 1.00E-04 | > 1.00E-04 |
| SK-OV-3 | 4.43E-05 | > 1.00E-04 | > 1.00E-04 |

| Nierenkrebs | | | |
|---|---|---|---|
| 768-0 | > 1.00E-04 | > 1.00E-04 | > 1.00E-04 |
| ACHN | < 1.00E-08 | > 1.00E-04 | > 1.00E-04 |
| CAKI-1 | 1.44E-06 | > 1.00E-04 | > 1.00E-04 |
| RXF 393 | 5.12E-06 | > 1.00E-04 | > 1.00E-04 |
| SN12C | 1.73E-07 | > 1.00E-04 | > 1.00E-04 |
| TK-10 | 6.25E-06 | > 1.00E-04 | > 1.00E-04 |

| Prostatakrebs | | | |
|---|---|---|---|
| PC-3 | 2.28E-06 | > 1.00E-04 | > 1.00E-04 |
| DU-145 | 1.58E-07 | > 1.00E-04 | > 1.00E-04 |

| Brustkrebs | | | |
|---|---|---|---|
| NCI/ADR-RES | 6.95E-07 | > 1.00E-04 | > 1.00E-04 |
| MDA-MB-231/ATCC | 9.55E-05 | > 1.00E-04 | > 1.00E-04 |
| HS 578T | 7.32E-07 | > 1.00E-04 | > 1.00E-04 |
| MDA-MB-435 | 4.76E-06 | > 1.00E-04 | > 1.00E-04 |
| MDA-N | 2.54E-05 | > 1.00E-04 | > 1.00E-04 |
| BT-549 | 4.15E-06 | > 1.00E-04 | > 1.00E-04 |
| T-47D | 1.42E-06 | > 1.00E-04 | > 1.00E-04 |

Aufgrund obiger in vitro Testergebnisse eignet sich dieser Wirkstoff bevorzugt zur Therapie von Tumoren der Lunge, des Zentralnervensystems, der Niere, der Brust sowie für die Behandlung von Melanomen.

## Patentansprüche

1. Glycerylnucleotide der Formel la 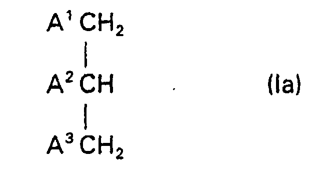 worin
a) einer der Reste A¹, A² und A³ für ein Wasserstoffatom, oder einen Rest, ausgewählt unter Hydroxyl, Mecapto, Alkyl, Alkenyl, Polyoxyalkenyl, Aryl, Acyl, Alkyloxy, Alkenyloxy, Polyoxyalkenyloxy, Acyloxy, Aryloxy, Alkylthio, Alkenylthio, Acylthio oder Arylthio stehen, wobei die Alkyl-, Alkenyl- und Acyl gegebenenfalls mit 1 bis 3 Aryl- Resten substituiert sind; und
b1) zwei der verbleibenden Reste A¹, A² und A³ für zwei voneinander verschiedene Nucleosidgruppen stehen, welche jeweils über eine physiologisch spaltbare phosphorhaltige Brückengruppe mit dem Kohlenstoffatom der Glycerylkette verbunden sind; oder
b2) einer der verbleibenden Reste A¹, A² und A³ für eine Nucleosidgruppe und der andere der verbleibenden Reste für eine Hydroxycarbonylgruppe steht, welche jeweils über eine physiologisch spaltbare phosphorhaltige Brückengruppe mit dem Kohlenstoffatom der Glycerylkette verbunden sind;
wobei wenigstens eine der Nucleosidgruppen keine natürlich vorkommende Nucleosidgruppe ist, welche in ihrem Basenteil gegebenenfalls substituiert ist mit einem oder mehreren Resten, ausgewählt unter Hydroxyl, Amino, Halogen, Alkyl, Alkenyl, Polyoxyalkenyl, Aryl, Acyl, Alkyloxy, Alkenyloxy, Polyoxyalkenyloxy, Acyloxy, Aryloxy, Alkylthio, Alkenylthio, Acylthio oder Arylthio, wobei die Alkyl-, Alkenyl- und Acyl gegebenenfalls mit 1 bis 3 ArylResten oder Halogenatomen substituiert sind; und welche in ihrem Kohlenhydratteil gegebenenfalls ein- oder mehrfach substituiert ist mit Substituenten, ausgewählt unter Wasserstoff, Halogen, Hydroxyl, Ethinyl oder Azido, gegebenenfalls ein Heteroatom, ausgewählt unter S , N und O anstelle eines Kohlenstoffatoms aufweist und gegebenenfalls eine oder zwei nichtbenachbarte C=C-Doppelbindungen enthält;
in racemischer oder enantiomerenreiner Form sowie die pharmazeutisch verträglichen Salze dieser Verbindungen.

2. Verbindung nach Anspruch 1, worin Nucleosidgruppen unabhängig voneinander über eine Brückengruppe ausgewählt unter -OP(OZ)(O)O-, -SP(OZ)(O)O-, -OP(OZ)(S)O- oder -SP(OZ)(S)O- und die Hydroxycarbonylgruppe über eine Brückengruppe ausgewählt unter -OP(OZ)(O)-, -SP(OZ)(O)- oder -SP(OZ)(S)-, worin Z für ein Proton oder ein pharmazeutisch vertägliches Kation steht, mit dem Glycerylrest verbunden sind.

3. Verbindungen nach Anspruch 1 oder 2, wobei A¹, A² und A³ so gewählt sind, das dem Molekül ein amphiphiler Charakter verliehen wird.

4. Verbindungen nach einem der vorherigen Ansprüche der Formel I, 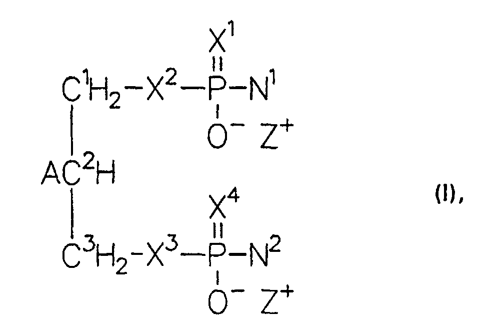 in racemischer und enantiomeren reiner Form,
worin
der Rest A und die beiden Phosphor-haltigen Reste in beliebiger Reihenfolge mit den C-Atomen C¹, C² und C³ des Glyceringerüstes verbunden sind;
X¹, X², X³ und X⁴ gleich oder verschieden sind und unabhängig voneinander für Sauerstoff oder Schwefel stehen;
A für Alkyl, Hydroxyl, Mercapto, Alkyl- oder Acyl-substituiertes Hydroxyl oder Mercapto steht, wobei die Alkyl- oder Acylreste linear oder verzweigt sind, 1 bis 24 C-Atome besitzen, gegebenenfalls bis zu 2 Doppelbindungen aufweisen und gegebenenfalls durch 1 bis 3 aromatische Reste substituiert sind;
Z für Wasserstoff oder das entsprechende Salz der sauren Form dieser Verbindung steht;
einer der Reste N¹ oder N² für Hydroxycarbonyl oder dessen Salzform steht und der andere Rest davon für den Rest eines D- oder L- konfigurierten Nucleosidderivats steht; oder beide Reste N¹ und N² voneinander verschieden sind und jeweils für einen Rest eines D- oder L-konfigurierten Nucleosidderivats stehen, wobei die Nukleosidreste ausgewählt sind unter Resten der allgemeinen Formel II, III und IV 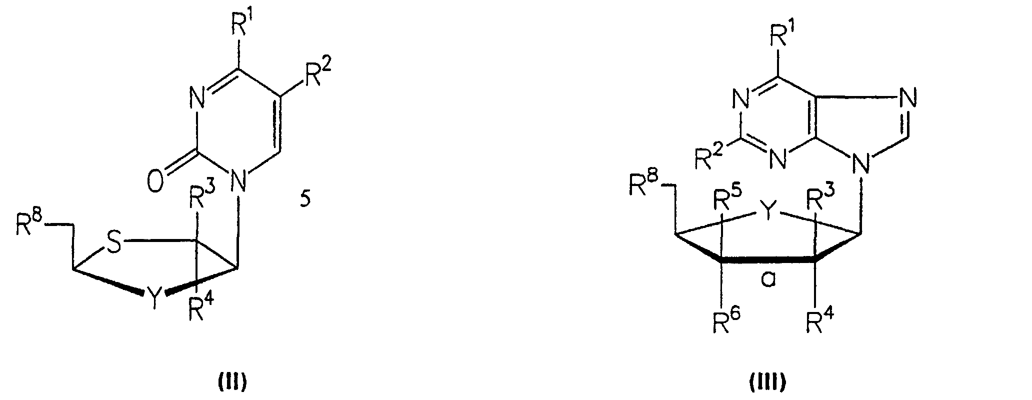 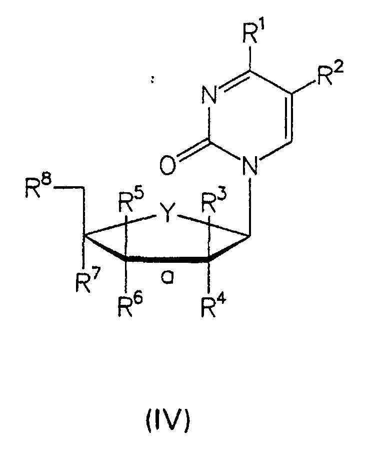 oder den tautomeren Formen davon, worin
Y für Sauerstoff oder Schwefel steht;
R¹ eine Hydroxyl-, Amino-, acylierte, alkylierte oder durch Polyoxyethylen substituierte Aminogruppe bedeutet, deren Acyl- oder Alkylrest linear oder verzweigt ist, 1 bis 24 C-Atome aufweist und gegebenenfalls bis zu 2 Doppelbindungen aufweist und durch einen aromatischen Rest substituiert sein kann;
R² für Wasserstoff, Halogen, eine Amino- oder Hydroxylgruppe, einen Bromvinyl- oder einen linearen oder verzweigten C₁-C₂₄ Alkylrest steht; und
R³, R⁴, R⁵, R⁶, R⁷ und R⁸ gleich oder verschieden sind und für Wasserstoff, Halogen, Hydroxyl, Ethinyl und Azido stehen; mit der Maßgabe, daß einer der Reste R³ bis R⁸ für ein Sauerstoffatom steht, über welches der Nucleosidderivat-Rest mit dem Phosphoratom verbunden ist, und zwei vicinale Reste R³ bis R⁶ dann entfallen, wenn in Position a eine C = C-Doppelbindung vorliegt.

5. Verbindungen nach Anspruch 4, worin
X¹, X², X³ und X⁴ ein Sauerstoffatom bedeuten;
das C¹-Atom des Glyceringerüstes der Formel I mit dem Rest A verbunden ist, wobei A für Hydroxyl, Octadecyl, Octadecyloxy, Docosyloxy oder Behenoyloxy, Palmitoyl oder Oleoyl steht;
das C²-Atom des Glyceringerüstes der Formel I über eine Phosphodiesterbrücke mit N² verbunden ist, wobei N² für einen Nucleosidderivat-Rest der Formel II, III oder IV steht, worin
Y für ein Sauerstoffatom steht;
R¹ für eine Hydroxyl-, Amino-, Octadecylamino-, Docosylamino-, Palmitoylamino-, Oleoylamino- oder Behenoyiaminogruppe steht;
R² für Methyl, Ethyl, Wasserstoff oder Halogen steht;
R³ bis R⁸ die oben angegebenen Bedeutungn besitzen, wobei einer der
Reste R³, R⁴, R⁵, R⁶ und R⁸ ein Sauerstoffatom darstellt, über das der Nucleosidderivat-Rest N² mit dem Phosphoratom verbunden ist,
und
das C³-Atom des Glyceringerüstes der Formel I mit dem Hydroxycarbonylphosphonatrest in seiner freien oder Salzform verbunden ist.

6. Verbindungen nach Anspruch 5, worin N² für einen Nucleosidderivatrest der Formel II oder IV steht, worin
R¹ eine Amino-, Palmitoylamino- oder Hydroxylgruppe bedeutet;
R² Wasserstoff, Methyl oder Ethyl bedeutet;
R³, R⁴, R⁵ und R⁷ für Wasserstoff stehen;
R⁶ Wasserstoff, Fluor oder Azido bedeuten, und
R⁸ ein Sauerstoffatom darstellt, über das N² mit dem Phosphoratom verbunden ist.

7. Verbindungen nach Anspruch 4, worin
X¹, X², X³ und X⁴ ein Sauerstoffatom bedeuten;
A für Palmitoyloxy-, Oleoyoxyl- und Octadecyloxy steht und mit dem C²-Atom des Glyceringerüstes der Formel I verbunden ist und
N¹ und N² verschieden sind und für einen Nucleosidderivat-Rest der Formeln II, III und IV stehen.

8. Verbindungen nach Anspruch 7, worin N¹ worin für einen L-konfigurierten Nucleosidderivat-Rest der Formel II steht,
worin
R¹ eine Amino- oder Palmitoylaminogruppe bedeutet;
R², R³ und R⁴ für Wasserstoff stehen, und
R⁸ ein Sauerstoffatom darstellt, über das N¹ mit dem Phosphoratom in Position C¹ oder C³ des Glyceryldiphosphatgerüstes der Formel I verbunden ist; und N² für einen Nucleosidderivat-Rest der Formel III oder IV steht.

9. Verbindungen nach Anspruch 7, worin N² einen Nucleosidderivat-Rest der Formel IV darstellt, worin
R¹ für eine Amino-, Palmitoylamino- oder Hydroxylgruppe steht;
R² für Wasserstoff oder Methyl steht;
R³, R⁴, R⁵ und R⁷ für Wasserstoff stehen;
R⁶ für Wasserstoff, Fluor oder Azido steht, und
R⁸ für ein Sauerstoffatom steht, über das N² mit dem Phosphoratom in Position C¹ oder C³ des Glyceryldiphosphatgerüstes der Formel I verbunden ist.

10. Verbindungen nach Anspruch 7, worin N¹ für einen Nucleosidderivat-Rest der Formel II oder IV steht, und
N² für einen Nucleosidderivat-Rest der Formel III steht,
worin jeweils
R¹ für Hydroxyl steht;
R² bis R⁷ für Wasserstoff stehen; und
R⁸ für ein Sauerstoffatom steht, über das N¹ bzw. N² mit dem Glycerin-1,3-diphosphatgerüst der Formel I verbunden ist.

11. Verbindungen nach Anspruch 7, worin
N¹ und N² für einen Nucleosidderivat-Rest der Formel IV stehen,
worin in N¹
R¹ für eine Amino-, Palmitoylamino- oder eine Octadecylaminogruppe steht;
R², R⁵ und R⁷ für Wasserstoff stehen;
R³, R⁴ und R⁶ gleich oder verschieden sind und für Wasserstoff, Hydroxyl oder Fluor stehen;
und worin in N²
R¹ für eine Hydroxylgruppe steht;
R² für Methyl oder Halogen steht;
R³, R⁴, R⁵ und R⁷ für Wasserstoff stehen, wobei gegebenenfalls zwei vicinale Reste davon entfallen, wenn in Position a eine Doppelbindung vorliegt;
R⁶ für Wasserstoff, Fluor, Azido oder Hydroxyl stehen; und
R⁸ in N¹ und N² jeweils ein Sauerstoffatom darstellt, über das N¹ und N² mit dem Glycerin-1,3-diphosphatgerüst verbunden sind.

12. Verbindungen nach Anspruch 11,
wobei in N¹ und N²
R¹ gleich oder verschieden ist und für eine Hydroxyl- oder Aminogruppe steht;
R² gleich oder verschieden ist und für Wassserstoff oder Methyl steht;
R⁴, R⁵ und R⁷ für Wasserstoff stehen;
R⁶ in N¹ für Azido und in N² für Fluor steht; und
R⁸ das Sauerstoffatom bedeutet, über das N¹ und N² mit dem Glycerin-1,3-diphosphatgerüst verbunden ist.

13. Verbindungen nach Anpruch 11,
worin in N¹,
R¹ für eine Amino- der Palmitoylaminogruppe steht,
R² und R⁷ für Wasserstoff stehen,
R³ und R⁴ für Fluor, Wasserstoff oder Hydroxyl stehen,
R⁵ für Wasserstoff oder einen Ethinylrest steht,
R⁶ für Hydroxyl steht, und
worin in N²,
R¹ für Hydroxyl steht,
R² für Fluor steht,
R³, R⁴, R⁵ und R⁷ für Wasserstoff stehen;
R⁶ für Hydroxyl steht, und
R⁸ in N¹ und N² jeweils ein Sauerstoffatom darstellt, über N¹ und N² mit dem Glycerin-1,3-diphosphatgerüst verbunden sind.

14. Verbindungen nach Anspruch 1, ausgewählt unter
a) 3'-Azido-2',3'-didesoxythymidylyl-(5'→2)-1-O-octadeyl-rac-glycerol-3-(hydroxycarbonyl)-phosphonat
b) Arabinocytidylyl-(5'→1)-2-O-octadecyl-rac-glycerylyl-(3→5')-5-fluoro-2'-desoxyuridin
c) N⁴-Palmitoyl-3'-thia-2',3'-didesoxycytidylyl-(5'→1)-2-O-octadecyl-racglycerylyl-(3→5')-3'-azido-2',3'-didesoxythymidin
d) 3'-Azido-2',3'-didesoxythymidylyl-(5'→1)-2-O-octadecyl-rac-glycerylyl-(3→5')-N⁴-palmitoyl-2',3'-didesoxycytidin
e) N⁴-Palmitoyl-2',3'-didesoxycytidylyl-(5'→1)-2-O-palmitoyl-racglycerylyl-(3→5')-3'-azido-2',3'-didesoxythymidin
f) 3'-Azido-2',3'-didesoxythymidylyl-(5'→1)-2-O-octadecyl-rac-glycerylyl-(3→5')-2',3'-didesoxyinosin
g) 3'-Fluoro-2',3'-didesoxythymidylyl-(5'→1)-2-O-octadecyl-rac-glycerylyl-(3→5')-3'-azido-2',3'-didesoxythymidin;
h) 2',3'-Didesoxycytidylyl-(5'→1)-rac-glycerylyl-(3→5')-5-fluoro-2'desoxyuridin.

15. Pharmazeutisches Mittel, enthaltend mindestens eine Verbindung nach einem der vorhergehenden Ansprüche in einem pharmazeutisch verträglichen Träger oder Verdünnungsmittel, gegebenenfalls in Verbindung mit einem pharmazeutisch verträglichen Formulierungsmittel oder eingebaut in Liposomen oder Nanopartikeln.

16. Verwendung einer Verbindung nach einem der Ansprüche 1 bis 14 zur Herstellung eines pharmazeutischen Mittels zur Behandlung von Infektionskrankheiten und /oder Krebserkrankungen

17. Verfahren zur Herstellung einer Verbindung der Formel la nach Anspruch 1, **dadurch gekennzeichnet, daß** man eine Verbindung der Formel Ib 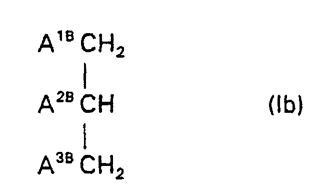 worin
einer der Reste A^{1B}, A^{2B} und A^{3B} für eine Hydroxy-, Mercapto-, Hydrogenphosphonat- oder Thiohydrogenphosphonatgruppe steht, und die beiden anderen Reste die oben für A¹, A² und A³ angegebenen Bedeutungen besitzen, wobei wenigstens einer der beiden Reste für eine Nucleosidgruppe gemäß obiger Definition steht,
a) in Gegenwart eines Säurechlorids mit einem Nukleosidderivat kondensiert, wobei das Nukleosidderivat zusätzlich eine Hydrogenphosphonat- oder Thiohydrogenphosphonatgruppe trägt, wenn einer der Reste A^{1B}, A^{2B} und A^{3B} für eine Hydroxy-, oder Mercapto-Gruppe steht; und das erhaltenen Produkt oxidiert; oder
b) mit (Ethoxycarbonyl)dichlorophosphonat umsetzt und anschließend die Säurechlorid- und Ethoxygruppen alkalisch hydrolysiert.

18. Verfahren nach Anspruch 17, **dadurch gekennzeichnet, daß** man eine Verbindung der Formel V 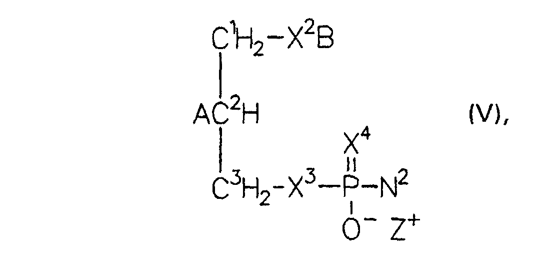 worin A, X³, X⁴, Z und N² die in den Ansprüchen 4 und 5 angegebenen Bedeutungen haben, und X²B für eine Hydroxyl- oder Mercaptogruppe steht, mit (Ethoxycarbonyl)dichlorophosphonat umsetzt, den Reaktionsansatz gegebenenfalls chromatographisch aufreinigt und anschließend die Chloridund Ethoxygruppe gegen eine Hydroxylgruppe oder die entsprechende Salzform tauscht.

19. Verfahren nach Anspruch 17, **dadurch gekennzeichnet, daß** man eine Verbindung der Formel V, 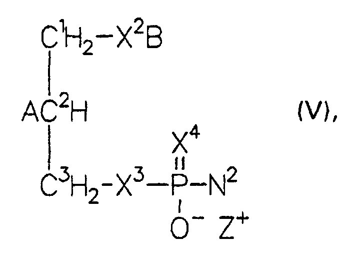 worin A, X³, X⁴, Z und N² die in den Ansprüchen 6-13 angegebenen Bedeutungen haben und X²B für eine Hydroxy-, Mercapto-, Hydrogenphosphonat- oder Thiohydrogenphosphonatgruppe steht, mit einem Nucleosidderivat der Formel II, III oder IV, 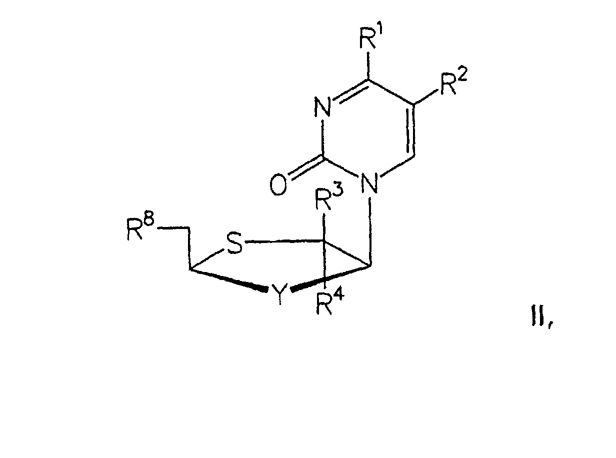 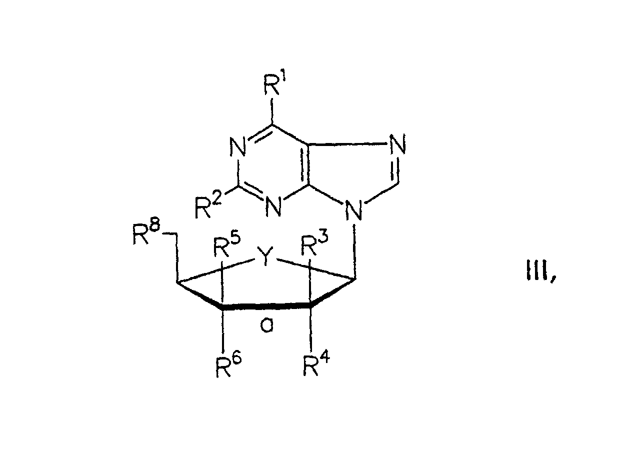 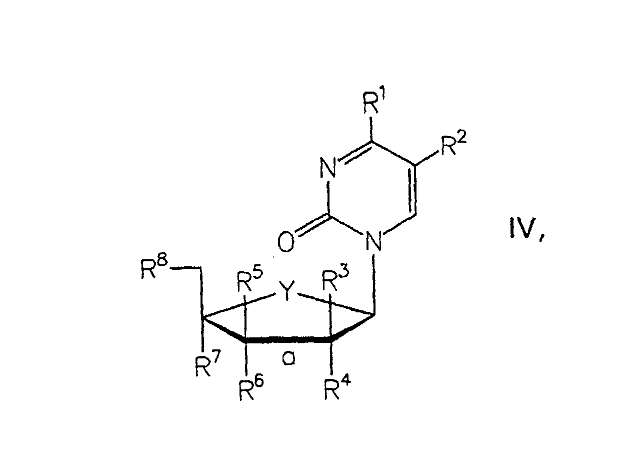 worin
R¹ bis R⁸ die in den Ansprüchen 4-13 angegebenen Bedeutungen haben, und zusätzlich R⁸ auch für 4-Mono-, 4,4'-Dimethoxytriphenylmethoxy, Hydrogenphosphonat oder Thiohydrogenphosphonat stehen kann;
R³, R⁴, R⁵ und R⁶ auch für einen linearen oder verzweigten Carboxylrest stehen, der 1 - 24 C-Atome aufweist und durch einen Phenylrest substituiert sein kann; mit der Maßgabe, daß einer der Reste R³, R⁴, R⁵, R⁶ und R⁸ für Hydrogenphosphonat oder Thiohydrogenphosphonat steht, wenn X²B in der Verbindung der Formel V für Hydroxyl oder Thiol steht;
in Gegenwart eines Säurechlorids kondensiert und anschließend oxidiert; das Reaktionsgemisch gegebenenfalls chromatographischer aufarbeitet und gegebenenfalls vorhanden 4-Mono- oder 4,4'-Dimethoxytriphenylmethylgruppen gegen Hydroxylgruppen tauscht und gewünschtenfalls Acylreste hydrolytisch in Mercapto-, Hydroxyl- und/oder Aminogruppen überführt.

## Claims

1. Glycerylnucleotides of formula 1a 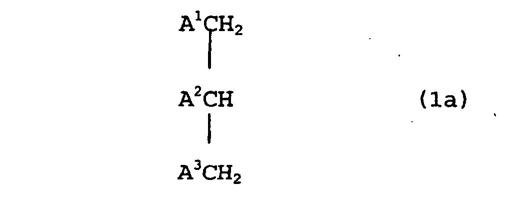 wherein
a) one of the groups A¹, A² and A³ denotes a hydrogen atom or a group selected from among hydroxyl, mercapto, alkyl, alkenyl, polyoxyalkenyl, aryl, acyl, alkyloxy, alkenyloxy, polyoxyalkenyloxy, acyloxy, aryloxy, alkylthio, alkenylthio, acylthio or arylthio, wherein the alkyl, alkenyl and acyl are optionally substituted by 1 to 3 aryl groups; and
b1) two of the remaining groups A¹, A² and A³ denote two different nucleoside groups, each linked to the carbon atom of the glyceryl chain via a physiologically cleavable bridging group containing phosphorus; or
b2) one of the remaining groups A¹, A² and A³ denotes a nucleoside group and the other of the remaining groups denotes a hydroxycarbonyl group, each of which is linked to the carbon atom of the glyceryl chain via a physiologically cleavable bridging group containing phosphorus;
at least one of the nucleoside groups being a nucleoside group which does not occur naturally, which is optionally substituted in its base moiety with one or more groups selected from among hydroxyl, amino, halogen, alkyl, alkenyl, polyoxyalkenyl, aryl, acyl, alkyloxy, alkenyloxy, polyoxyalkenyloxy, acyloxy, aryloxy, alkylthio, alkenylthio, acylthio or arylthio, wherein the alkyl, alkenyl and acyl are optionally substituted by 1 to 3 aryl groups or halogen atoms; and which, is optionally mono- or polysubstituted in its carbohydrate moiety by substituents selected from among hydrogen, halogen, hydroxyl, ethynyl or azido, optionally comprises a heteroatom selected from among S, N and O instead of a carbon atom and optionally contains 1 or 2 non-adjacent C=C double bonds;
in racemic or enantiomerically pure form, and the pharmaceutically acceptable salts of these compounds.

2. Compound according to claim 1, wherein nucleoside groups independently of one another are linked to the glyceryl group via a bridging group selected from among -OP(OZ)(O)O-, -SP(OZ)(O)O-, -OP(OZ)(S)O- or -SP(OZ)(S)O- and the hydroxycarbonyl group is linked to the glyceryl group via a bridging group selected from among -OP(OZ)(O)-, -SP(OZ)(O)- or -SP(OZ)(S)-, where Z represents a proton or a pharmaceutically acceptable cation.

3. Compounds according to claim 1 or 2, wherein A¹, A² and A³ are selected so as to impart an amphiphilic nature to the molecule.

4. Compounds according to one of the preceding claims, of formula I 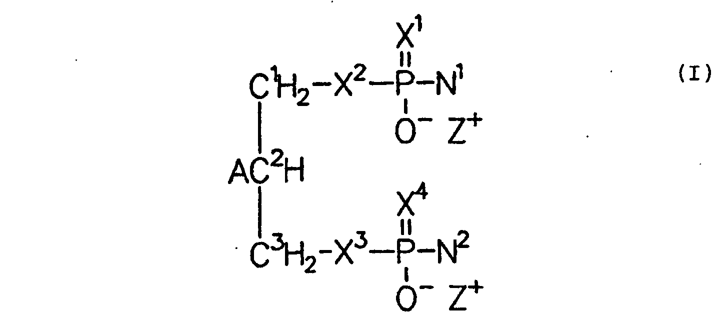 in racemic and enantiomerically pure form,
wherein
the group A and the two groups containing phosphorus are attached to the C-atoms C¹, C² and C³ of the glycerol structure in any desired sequence;
X¹, X², X³ and X⁴ may be identical or different and independently of one another represent oxygen or sulphur;
A denotes alkyl, hydroxyl, mercapto, alkyl- or acyl-substituted hydroxyl or mercapto, wherein the alkyl or acyl groups are straight-chain or branched, have 1 to 24 carbon atoms, optionally comprise up to 2 double bonds and are optionally substituted by 1 to 3 aromatic groups;
Z denotes hydrogen or the corresponding salt of the acid form of this compound;
one of the groups N¹ or N² denotes hydroxycarbonyl or the salt form thereof and the other group denotes the group of a nucleoside derivative in D- or L- configuration; or the two groups N¹ and N² are different from each other and each represent a group of a nucleoside derivative in D- or L- configuration, the nucleoside groups being selected from among groups of general formula II, III and IV 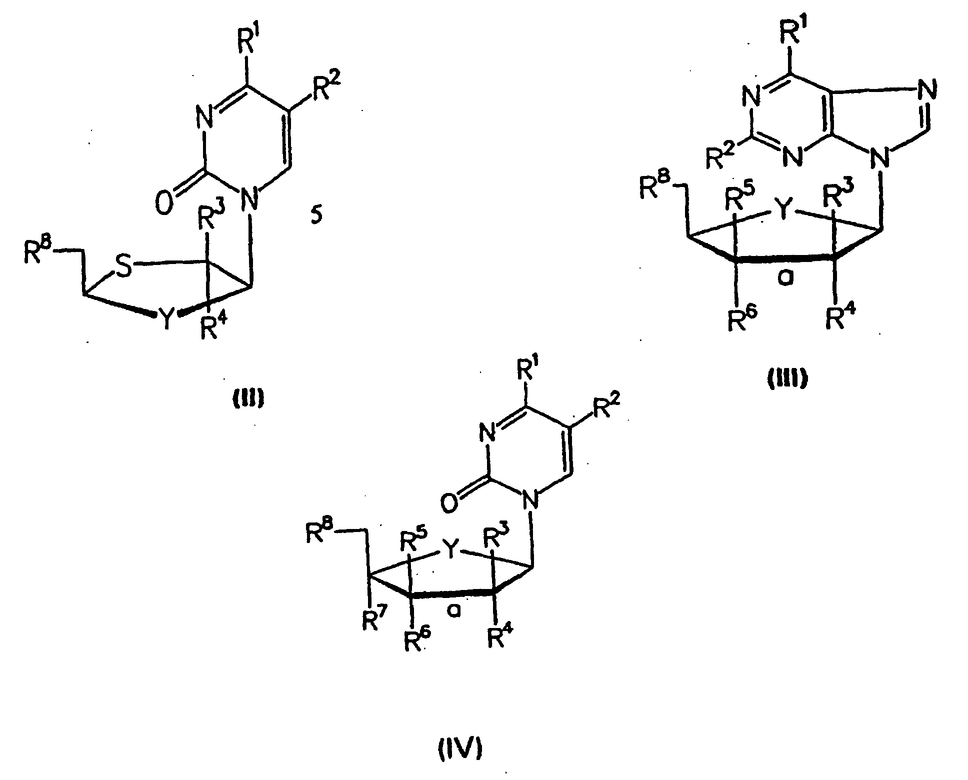 or the tautomeric forms thereof, wherein
Y denotes oxygen or sulphur;
R¹ denotes a hydroxyl, amino, acylated, alkylated or polyoxyethylene-substituted amino group, the acyl or alkyl group of which is straight-chain or branched, comprises 1 to 24 carbon atoms and optionally up to 2 double bonds and may be substituted by an aromatic group;
R² denotes hydrogen, halogen, an amino or hydroxyl group, a bromovinyl or a straight-chain or branched C₁-C₂₄ alkyl group;
and
R³, R⁴, R⁵, R⁶, R⁷ and R⁸ are identical or different and denote hydrogen, halogen, hydroxyl, ethynyl and azido; with the proviso that one of the groups R³ to R⁸ denotes an oxygen atom via which the nucleoside derivative group is attached to the phosphorus atom, and two vicinal groups R³ to R⁶ are omitted when there is a C=C double bond in position A.

5. Compounds according to claim 4, wherein
X¹, X², X³ and X⁴ denote an oxygen atom;
the C¹ atom of the glycerol structure of formula I is attached to the group A, where A denotes hydroxyl, octadecyl, octadecyloxy, docosyloxy or behenoyloxy, palmitoyl or oleoyl;
the C² atom of the glycerol structure of formula I is attached to N² via a phosphodiester bridge, where N² denotes a nucleoside derivative group of formula II, III or IV, wherein
Y denotes an oxygen atom;
R¹ denotes a hydroxyl, amino, octadecylamino, docosylamino, palmitoylamino, oleoylamino or behenoylamino group;
R² denotes methyl, ethyl, hydrogen or halogen;
R³ to R⁸ are as hereinbefore defined, one of the groups R³, R⁴, R⁵, R⁶ and R⁸ representing an oxygen atom via which the nucleoside derivative group N² is attached to the phosphorus atom,
and
the C³ atom of the glycerol structure of formula I is attached to the hydroxycarbonyl phosphonate group in its free or salt form.

6. Compounds according to claim 5, wherein
N² denotes a nucleoside derivative group of formula II or IV, wherein
R¹ denotes an amino, palmitoylamino or hydroxyl group;
R² denotes hydrogen, methyl or ethyl;
R³, R⁴, R⁵ and R⁷ denote hydrogen;
R⁶ denotes hydrogen, fluorine or azido, and
R⁸ denotes an oxygen atom via which the N² is attached to the phosphorus atom.

7. Compounds according to claim 4, wherein
X¹, X², X³ and X⁴ denote an oxygen atom;
A denotes palmitoyloxy, oleoyloxy and octadecyloxy and is attached to the C² atom of the glycerol structure of formula I and
N¹ and N² are different and represent a nucleoside derivative group of formulae II, III and IV.

8. Compounds according to claim 7, wherein
N¹ represents a nucleoside derivative group of formula II in the L-configuration, wherein
R¹ denotes an amino or palmitoylamino group;
R², R³ and R⁴ denote hydrogen, and
R⁸ denotes an oxygen atom by means of which N¹ is attached to the phosphorus atom in position C¹ or C³ of the glyceryldiphosphate structure in formula I;
and N² denotes a nucleoside derivative group of formula III or IV.

9. Compounds according to claim 7, wherein
N² denotes a nucleoside derivative group of formula IV, wherein
R¹ denotes an amino, palmitoylamino or hydroxyl group;
R² denotes hydrogen or methyl;
R³, R⁴, R⁵ and R⁷ denote hydrogen;
R⁶ denotes hydrogen, fluorine or azido, and
R⁸ denotes an oxygen atom via which N² is attached to the phosphorus atom in position C¹ or C³ of the glyceryldiphosphate structure of formula I.

10. Compounds according to claim 7, wherein
N¹ denotes a nucleoside derivative group of formula II or IV, and
N² denotes a nucleoside derivative group of formula III, wherein in each case
R¹ denotes hydroxyl;
R² to R⁷ denote hydrogen; and
R⁸ denotes an oxygen atom via which N¹ or N² is attached to the glycerol-1,3-diphosphate structure of formula I.

11. Compounds according to claim 7, wherein
N¹ and N² denote a nucleoside derivative group of formula IV, wherein in N¹
R¹ denotes an amino, palmitoylamino or octadecylamino group;
R², R⁵ and R⁷ denote hydrogen;
R³, R⁴ and R⁶ may be identical or different and denote hydrogen, hydroxyl or fluorine;
and wherein in N²
R¹ denotes a hydroxyl group;
R² denotes methyl or halogen;
R³, R⁴, R⁵ and R⁷ denote hydrogen, whilst optionally two vicinal groups are omitted if there is a double bond in position A;
R⁶ denotes hydrogen, fluorine, azido or hydroxyl; and
R⁸ in N¹ and N² in each case denotes an oxygen atom via which N¹ and N² are attached to the glycerol-1,3-diphosphate structure.

12. Compounds according to claim 11,
wherein in N¹ and N²
R¹ may be identical or different and denotes a hydroxyl or amino group;
R² may be identical or different and denotes hydrogen or methyl;
R⁴, R⁵ and R⁷ denote hydrogen;
R⁶ in N¹ denotes azido and in N² denotes fluorine; and
R⁸ denotes the oxygen atom via which N¹ and N² are attached to the glycerol 1,3-diphosphate structure.

13. Compounds according to claim 11,
wherein in N¹
R¹ denotes an amino of the palmitoylamino group,
R² and R⁷ denote hydrogen,
R³ and R⁴ denote fluorine, hydrogen or hydroxyl,
R⁵ denotes hydrogen or an ethynyl group,
R⁸ denotes hydroxyl, and
wherein in N²
R¹ denotes hydroxyl,
R² denotes fluorine,
R³, R⁴, R⁵ and R⁷ denote hydrogen;
R⁶ denotes hydroxyl, and
R⁸ in N¹ and N² denotes an oxygen atom via which N¹ and N² are attached to the glycerol 1,3-diphosphate structure.

14. Compounds according to claim 1, selected from among
a) 3'-azido-2',3'-didesoxythymidylyl-(5'→2)-1-O-octadecyl-rac-glycerol-3-(hydroxycarbonyl)-phosphonate
b) arabinocytidylyl- (5'→1) -2-O-octadecyl-rac-glycerylyl- (3→5')-5-fluoro-2'-desoxyuridine
c) N⁴-palmitoyl-3'-thia-2',3'-didesoxycytidylyl-(5'→1)-2-O-octadecyl-rac-glycerylyl-(3→5')-3'-azido-2',3'-didesoxythymidine
d) 3'-azido-2',3'-didesoxythymidylyl-(5'→1)-2-O-octadecyl-rac-glycerylyl-(3→5')-N⁴-palmitoyl-2',3'-didesoxycytidine
e) N⁴-palmitoyl-2',3'-didesoxycytidylyl-(5'→1)-2-O-palmitoyl-rac-glycerylyl-(3→5')-3'-azido-2',3'-didesoxythymidine
f) 3'-azido-2',3'-didesoxythymidylyl-(5'→1)-2-O-octadecyl-rac-glycerylyl-(3→5')-2',3'-didesoxyinosine
g) 3'-fluoro-2',3'-didesoxythymidylyl-(5'→1)-2-O-octadecyl-rac-glycerylyl-(3→5')-3'-azido-2',3'-didesoxythymidine;
h) 2',3'-didesoxycytidylyl-(5'→1)-rac-glycerylyl-(3→5')-5-fluoro-2'-desoxyuridine.

15. Pharmaceutical composition containing at least one compound according to one of the preceding claims in a pharmaceutically acceptable carrier or diluent, optionally combined with a pharmaceutically acceptable formulating agent or incorporated in liposomes or nanoparticles.

16. Use of a compound according to one of claims 1 to 14 for the preparation of a pharmaceutical composition for treating infectious diseases and/or cancers.

17. Process for preparing a compound of formula Ia according to claim 1, **characterised in that** a compound of formula 1b 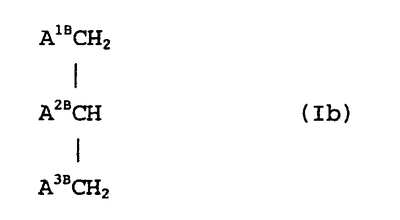 wherein
one of the groups A^{1B}, A^{2B} and A^{3B} denotes a hydroxy, mercapto, hydrogen phosphonate or thiohydrogen phosphonate group, and the other two groups have the meanings given for A¹, A² and A³ hereinbefore, at least one of the two groups representing a nucleoside group as hereinbefore defined,
a) is condensed in the presence of an acid chloride with a nucleoside derivative, while the nucleoside derivative additionally carries a hydrogen phosphonate or thiohydrogen phosphonate group if one of the groups A^{1B}, A^{2B} and A^{3B} denotes a hydroxy or mercapto group; and the resulting product is oxidised; or
b) is reacted with (ethoxycarbonyl)dichlorophosphonate and subsequently the acid chloride and ethoxy groups are hydrolysed under alkaline conditions.

18. Process according to claim 17, **characterised in that** a compound of formula V 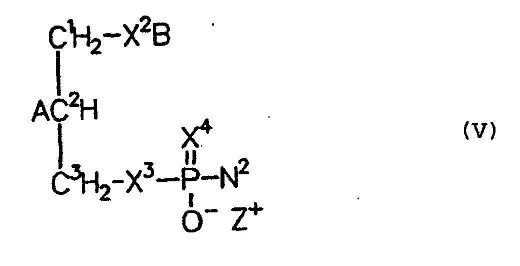 wherein A, X³, X⁴, Z and N² have the meanings given in claims 4 and 5 and X²B denotes a hydroxyl or mercapto group, is reacted with (ethoxycarbonyl) dichlorophosphonate, the reaction mixture is optionally purified by chromatography and subsequently the chloride and ethoxy group are replaced by a hydroxyl group or the corresponding salt form.

19. Process according to claim 17, **characterised in that** a compound of formula V 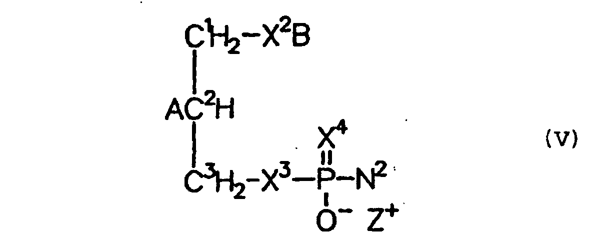 wherein A, X³, X⁴, Z and N² have the meanings given in claims 6-13 and X²B denotes a hydroxy, mercapto, hydrogen phosphonate or thiohydrogen phosphonate group, is condensed in the presence of an acid chloride with a nucleoside derivative of formula II, III or IV, 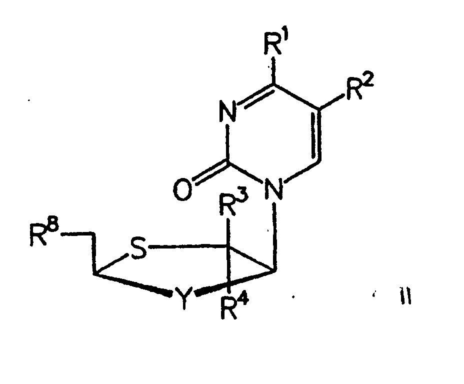 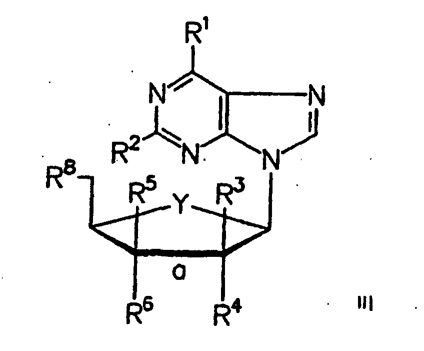 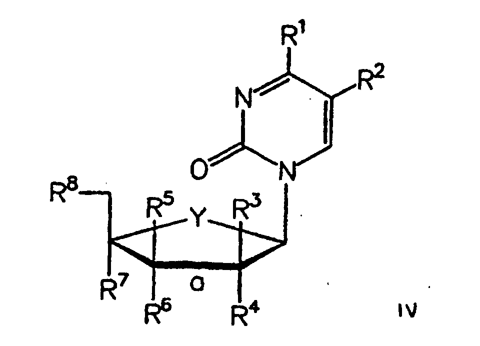 wherein
R¹ to R⁸ have the meanings given in claims 4-13, and additionally R⁸ may also denote 4-mono-, 4,4'-dimethoxytriphenylmethoxy, hydrogen phosphonate or thiohydrogen phosphonate;
R³, R⁴, R⁵ and R⁶ also denote a straight-chain or branched carboxyl group which has 1-24 carbon atoms and may be substituted by a phenyl group; with the proviso that one of the groups R³, R⁴, R⁵, R⁶ and R⁸ denotes hydrogen phosphonate or thiohydrogen phosphonate if X²B in the compound of formula V denotes hydroxyl or thiol;
and is subsequently oxidised;
the reaction mixture is optionally worked up by chromatographic methods and any 4-mono- or 4,4'-dimethoxytriphenylmethyl groups present are replaced by hydroxyl groups and if desired acyl groups are converted by hydrolysis into mercapto, hydroxyl and/or amino groups.

## Revendications

1. Nucléotide de glycéryle de formule I a : 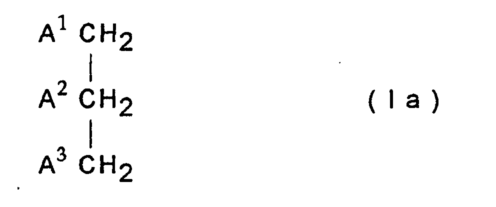 où :
a) l'un des radicaux A¹, A², A³ désigne un atome d'hydrogène, ou un radical choisi parmi les groupes hydroxyle, mercapto, alkyle (ou alcoyle), alcényle, polyoxyalcényle, aryle, acyle, alkyloxy, alcényloxy, polyoxyalcényloxy, acyloxy, aryloxy, alkylthio, alcénylthio, acylthio ou arylthio, les groupes alkyle, alcényle et acyle étant éventuellement substitués par 1 à 3 radicaux aryle,
b1) et soit deux des radicaux A¹, A², A³ restants sont des groupes nucléoside différents l'un de l'autre, qui sont liés chacun à l'atome de carbone du noyau glycéryle par un pont phosphoré séparable physiologiquement,
b2) soit l'un des radicaux A¹, A², A³ restants désigne un groupe nucléoside et l'autre un groupe hydroxycarbonyle, chacun étant lié par un pont phosphoré séparable physiologiquement à l'atome de carbone du noyau glycéryle,
au moins l'un des groupes nucloside étant un groupe nucléoside n'existant pas dans la nature
dont la partie basique est éventuellement substituée par un ou plusieurs radicaux choisis parmi les groupes hydroxyle, amino, halogène, alkyle, alcényle, polyoxyalcényle, aryle, acyle, alkyloxy, alcényloxy, polyoxyalcényloxy, acyloxy, aryloxy, alkylthio, alcénylthio, acylthio ou arylthio, les groupes alkyle, alcényle et acyle étant éventuellement substitués par 1 à 3 radicaux aryle ou par un atome d'halogène,
et qui dans sa partie hydrocarbure, est éventuellement substitué une ou plusieurs fois par un substituant constitué par un hydrogène, un halogène, un groupe hydroxyle, éthynyle ou azido, qui présente éventuellement un hétéroatome choisi parmi S, N et O en remplacement d'un atome de carbone et qui comporte éventuellement une ou deux doubles liaisons C = C non contigües,
sous forme racémique ou énantiomère, ainsi que les sels pharmaceutiquement acceptables de ces composés.

2. Composé suivant la revendication 1, dans lequel les groupes nucléoside, indépendamment l'un de l'autre, sont liés au radical glycérylique, par un groupe formant pont qui est choisi parmi les groupes -OP(OZ)(O)O-, -SP(OZ)(O)O-, -OP(OZ)(S)O- ou -SP(OZ)(S)O-, tandis que les groupes hydrocarbonyle sont liés au radical glycérylique par un groupe formant pont qui est choisi parmi les groupes -OP(OZ)(O)-, -SP(OZ)(O)- ou -SP(OZ)(S)O-, où Z est un proton ou un cation pharmaceutiquement compatible.

3. Composés suivant la revendication 1 ou 2, dans lesquels A¹, A², A³ sont choisis de telle sorte que la molécule présente un caractère amphiphile.

4. Composés suivant l'une quelconque des revendications précédentes répondant à la formule I : 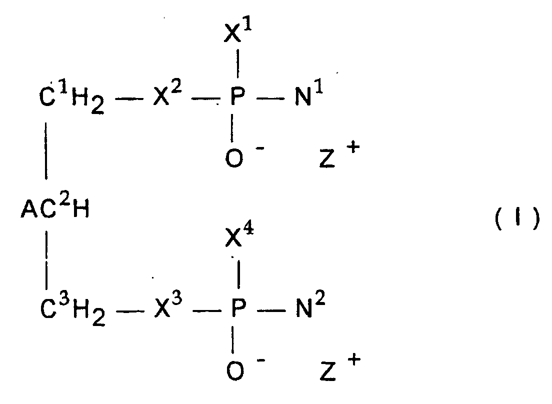 sous forme racémique ou sous forme d'énantiomère,
où :
le radical A et les deux radicaux phosphorés sont branchés sur l'un quelconque des atomes C en position C¹, C² ou C³ du radical glycérine,
X¹, X², X³ et X⁴ sont identiques ou différents et sont indépendamment l'un de l'autre l'oxygène ou le soufre,
A est un groupe alkyl, hydroxyle, mercapto, hydroxyle ou mercapto substitué alkyle ou acyle, dans lequel les radicaux alkyle ou acyle sont linéaires ou ramifiés, ledit groupe compportant de 1 à 24 atomes C, éventuellement jusqu'à deux doubles liaisons et étant éventuellement substitué par 1 à 3 radicaux aromatiques.
Z est l'hydrogène ou un sel correspondant de la forme acide du composé,
l'un des radicaux N¹ ou N² est un groupe hydroxycarbonyle ou sa forme saline et l'autre est un radical de dérivé de nucléoside de configuration D ou L, ou les deux radicaux N¹ ou N² sont différents l'un de l'autre et sont constitués chacun par un radical d'un dérivé nucléoside de configuration D ou L, le radical nucléoside étant un radical répondant à la formule générale II, III ou IV 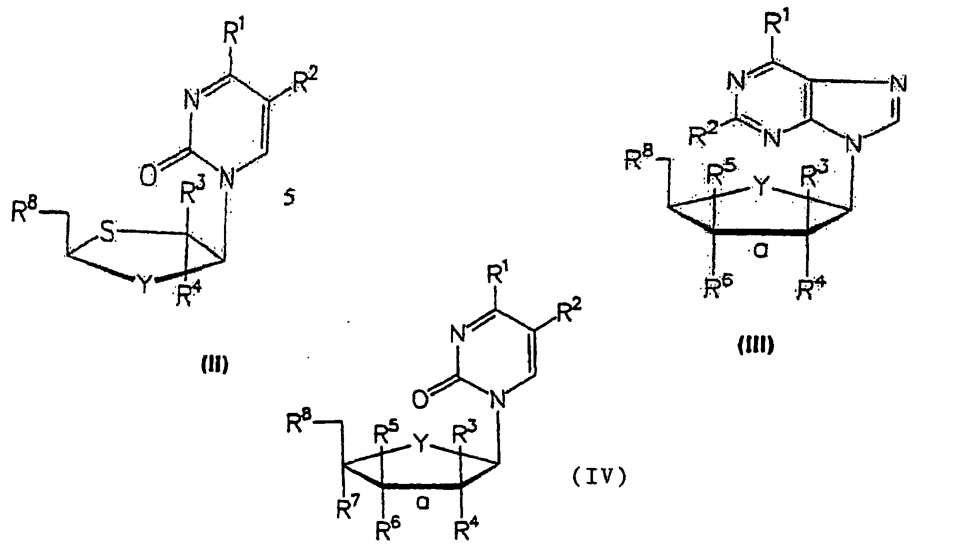
ou une forme tautomère d'un tel radical,
avec dans ces formules :
Y est l'oxygène ou le soufre,
R¹ désigne un groupe hydroxyle ou amino, ou un groupe amino substitué acyle, alkyle ou polyoxyéthylène, dont le radical acyle ou alkyle est linéaire ou ramifié, comporte de 1 à 24 atomes de carbone et éventuellement jusqu'à deux doubles liaisons, et peut être substitué par un radical aromatique,
R² représente l'oxygène, un halogène, un groupe amino ou hydroxyle, un radical bromovinyle ou alkyle en C₁ - C₂₄ linéaire ou ramifié,
et R³, R⁴, R⁵, R⁶, R⁷ et R⁸ sont identiques ou différents et représentent un groupe oxygène, halogène, hydroxyle, éthynyle ou azido, sous la réserve que l'un des radicaux R³ à R⁸ soit un atome d'oxygène par lequel le radical dérivé de nucléoside est lié à l'atome de phosphore et que deux radicaux voisins R³ à R⁸ disparaissent quand une double liaison C = C est présente en position a.

5. Composés suivant la revendication 4, dans lesquels X¹, X², X³ et X⁴ représentent chacun un atome d'oxygène.
l'atome C¹ du radical glycérine de formule I est lié au radical A, lequel est constitué par un groupe hydroxyle, octadécyle, octadécyloxy, docosyloxy ou béhénoyloxy, palmitoyle ou oléoyle,
l'atome C² du radical glycérine de formule I est lié à N² par un pont phospho-diester, N² étant un radical dérivé de nucléoside répondant à la formule II, III ou IV, où :
Y est l'oxygène,
R¹ désigne un groupe hydroxyle, amino, octadécylamino docosylamino, palmitoylamino, oléoylamino ou béhénoylamino,
R² représente un groupe méthyle, éthyle, hydrogène ou halogène,
R³ à R⁸ présentent les significations indiquées ci-dessus, l'un des radicaux R³, R⁴, R⁵, R⁶ et R⁸ est un atome d'oxygène par lequel le radical dérivé de nucléoside N² est lié à l'atome de phosphore,
et l'atome C³ du radical glycérine de formule I est lié au radical hydroxycarbonylphosphonate sous sa forme libre ou en forme salifiée.

6. Composés suivant la revendication 5, dans lesquels :
N² est un radical dérivé de nucléoside de formule II ou IV, où :
R¹ est un groupe amino, palmitoylamino ou hydroxyle,
R² est un oxygène, méthyle ou éthyle,
R³, R⁴, R⁵ et R⁷ sont l'hydrogène,
R⁶ est l'hydrogène, le fluor ou un groupe azido,
et R⁸ représente un atome d'oxygène qui est lié par N² avec l'atome de phosphore.

7. Composés suivant la revendication 4, dans lesquels :
X¹, X², X³ et X⁴ représentent chacun un atome d'oxygène,
A désigne un groupe palmitoyloxy, oléoyloxy ou octadécyloxy qui est fixé sur l'atome C² du radical glycérine de formule I,
N¹ et N² sont différents et représentent chacun un radical dérivé de nucléoside répondant à la formule II, III ou IV.

8. Composés suivant la revendication 7, dans lesquels :
N¹ est un radical dérivé de nucléoside en configuration L répondant à la formule II où :
R¹ est un groupe amino ou palmitoylamino,
R², R³ et R⁴ sont l'hydrogène,
R⁸ représente un atome d'oxygène qui est lié par N¹ avec l'atome de phosphore en position C¹ ou C³ du radical glycéryldiphosphate de la formule I,
et N² est un radical dérivé de nucléoside répondant à la formule III ou IV.

9. Composés suivant la revendication 7, dans lesquels :
N² est un radical dérivé de nucléoside de formule II ou IV, où :
R¹ est un radical amino, palmitoylamino ou hydroxyle,
R² est un oxygène ou un méthyle,
R³, R⁴, R⁵ et R⁷ sont l'hydrogène,
R⁶ est l'hydrogène, le fluor ou un groupe azido,
et R⁸ représente un atome d'oxygène qui est lié par N² avec l'atome de phosphore en position C¹ ou C³ du radical glycéryldiphosphate de la formule I.

10. Composés suivant la revendication 7, dans lesquels :
N¹ est un radical dérivé de nucléoside répondant à la formule II ou IV,
et N² est un radical dérivé de nucléoside de formule III,
avec dans chacun :
R¹ est un hydroxyle,
R² à R⁷ sont l'hydrogène,
et R⁸ représente un atome d'oxygène qui est lié par N¹ ou N² respectivement avec le radical glycérine-1,3-diphosphate répondant à la formule I.

11. Composés suivant la revendication 7, dans lesquels :
N¹ et N² sont chacun un radical dérivé de nucléoside de formule IV,
où dans N¹ :
R¹ est un radical amino, palmitoylamino ou octadécylamino,
R², R⁵ et R⁷ sont l'hydrogène,
R³, R⁴ et R⁶ sont identiques ou différents et représentent un hydrogène, hydroxyle ou un fluor,
et où dans N² :
R¹ est un radical hydroxyle,
R² est un méthyle ou un halogène,
R³, R⁴, R⁵ et R⁷ sont l'hydrogène, deux radicaux voisins R³ à R⁸ disparaissant éventuellement quand une double liaison C = C est présente en position a ,
R⁶ est l'hydrogène, le fluor, un groupe azido, ou un hydroxyle,
et R⁸ dans N¹ et dans N² représente un atome d'oxygène qui est lié par N¹ ou N² respectivement avec le radical glycérine-1,3-diphosphate.

12. Composés suivant la revendication 11, dans lesquels, dans N¹ et dans N² :
R¹ est identique ou différent et constitué par un radical hydroxyle ou un groupe amino,
R² est identique ou différent et constitué par l'hydrogène ou un groupe méthyle,
R⁴, R⁵ et R⁷ sont des atomes d'hydrogène,
R⁶ est un radical azido dans N¹ et un atome de fluor dans N²,
et R⁸ représente un atome d'oxygène qui est lié par N¹ et N² respectivement avec le radical glycérine-1,3-diphosphate.

13. Composés suivant la revendication 11, dans lesquels :
dans N¹ :
R¹ est un groupe amino ou palmitoylamino,
R² et R⁷ sont l'hydrogène,
R³ et R⁴ sont un fluor, un hydrogène ou un hydroxyle,
R⁵ est un hydrogène ou un radical éthynyle,
R⁶ est un hydroxyle,
et dans N² :
R¹ est un hydroxyle,
R² est un fluor,
R³, R⁴, R⁵ et R⁷ sont l'hydrogène,
R⁶ est un hydroxyle,
et R⁸ dans N¹ et dans N² représente un atome d'oxygène qui est lié par N¹ ou N² respectivement au radical glycérine-1,3-diphosphate.

14. Composés suivant la revendication 1, parmi les suivants
a) 3'-Azido-2',3'-didésoxythymidylyl-(5'→2)-1 -O-octadéyl-rac-glycérol-3-(hydroxycarbonyl)-phosphonate,
b) Arabinocytidylyl-(5'→1)-2-O-octadécyl-rac-glycérylyl-(3→5')-5-fluoro-2'-désoxyuridine,
c) N⁴-Palmitoyl-3'-thia-2',3'-didésoxycytidylyl-(5'→1)-2-O-octadécyl-rac-glycérylyl-(3→5')-3'-azido-2',3'-didésoxythymidine,
d) 3'-Azido-2',3'-didésoxythymidylyl-(5'→1)-2-O-octadécyl-rac-glycérylyl-(3→5')-N⁴-palmitoyl-2',3'-didésoxycytidine,
e) N⁴-Palmitoyl-2',3'-didésoxycytidylyl-(5'→1)-2-O-palmitoyl-rac-glycérylyl-(3→5')-3'-azido-2',3'-didésoxythymidine,
f) 3'-Azido-2',3'-didésoxythymidylyl-(5'→1)-2-O-octadécyl-rac-glycérylyl-(3→5')-2',3'-didésoxyinosine,
g) 3'-Fluoro-2',3'-didésoxythymidylyl-(5'→1)-2-O-octadécyl-rac-glycérylyl-(3→5')-3'-azido-2',3'-didésoxythymidine,
h) 2',3'-Didésoxycytidylyl-(5'→1)-rac-glycérylyl-(3→5')-5-fluoro-2'-désoxyuridine.

15. Produit pharmaceutique, comportant au moins un composé suivant l'une quelconque des revendications précédentes dans un milieu véhicule ou diluant pharmaceutiquement accceptable, éventuellement en combinaison avec une mileu de formulation pharmaceutiquement acceptable ou incorporé dans un liposome ou une nanoparticule.

16. Procédé de préparation d'un composé suivant la formule la de la revendication 1, **caractérisé en ce que**
l'on part d'un composé répondant à la formule Ib 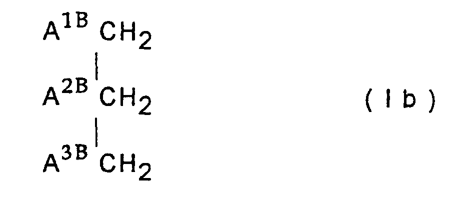 dans laquelle l'un des radicaux A^{1B}, A^{2B}, A^{3B} désigne un groupe hydroxyle, mercapto, hydrogéno-phosphonate ou thiohydrogéno-phosphonate et les deux autres ont les significations déjà indiquées pour les radicaux A¹, A², A³, l'un au moins des deux étant un groupe nucléoside suivant l'une des définitions ci-dessus,
a) que l'on condense en présence d'un chlorure d'acide avec un dérivé de nucléoside, ledit dérivé de nucléoside portant en outre un groupe hydrogénophosphonate ou thiohydrogéno-phosphonate, quand l'un l'un des radicaux A^{1B}, A^{2B}, A^{3B} est un hydroxyle ou un groupe mercapto, pour ensuite oxyder le produit obtenu,
b)ou que l'on fait réagir avec le (éthoxycarbonyl) dichlorophosphonate, avant de provoquer finalement une hydrolyse alcaline des groupes chlorure et éthoxy.

17. Utilisation d'un composé suivant l'une quelconque des revendications 1 à 14 pour la fabrication d'un produit pharmaceutique pour le traitement des maladies infectieuses et/ou virales.

18. Procédé suivant la revendication 17, **caractérisé en ce que** l'on fait réagir un composé de formule V 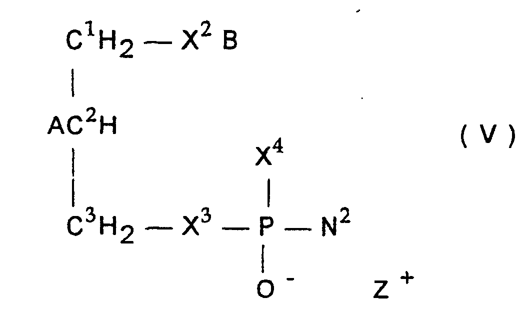 dans laquelle A, X³, X⁴, Z et N² ont les valeurs indiquées dans les revendications 4 et 5. et X^{2B} désigne un groupe hydroxyle ou mercapto,
avec le (éthoxycarbonyl)-dichlorophosphonate, on purifie si besoin le produit obtenu par chromatographie, et l'on termine en hydrolysant les groupes chlorure et éthoxy en un hydroxyle ou la forme saline correspondante.

19. Procédé suivant la revendication 17, **caractérisé en ce que** l'on part d'un composé de formule V 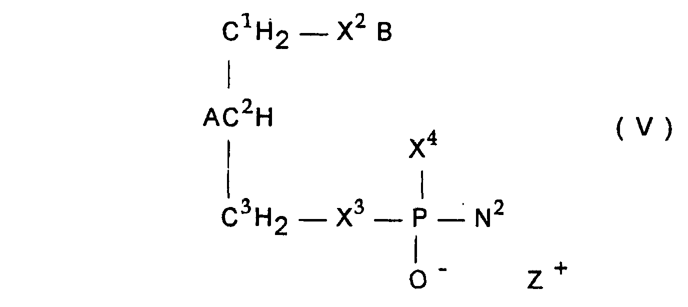 dans laquelle : A, X³, X⁴, Z et N² ont les valeurs indiquées dans les revendications 6 à 13 et X^{2B} désigne un groupe hydroxyle, mercapto, hydrogénophosphonate ou thiohydrogénophosponate,
dont on assure la condensation, en présence d'un chlorure d'acide, avec un dérivé de nucléoside suivant les formules II, III ou IV 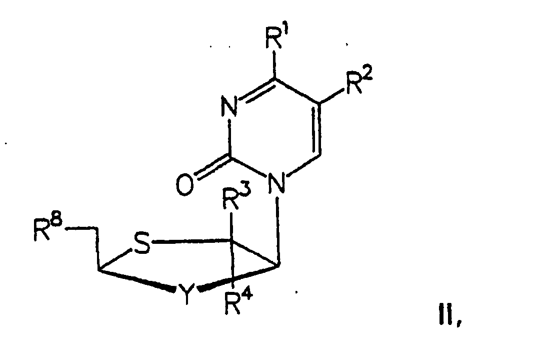 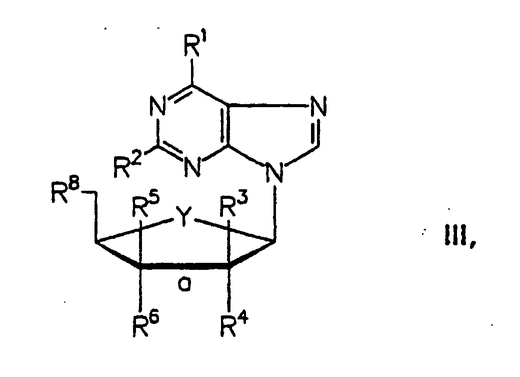 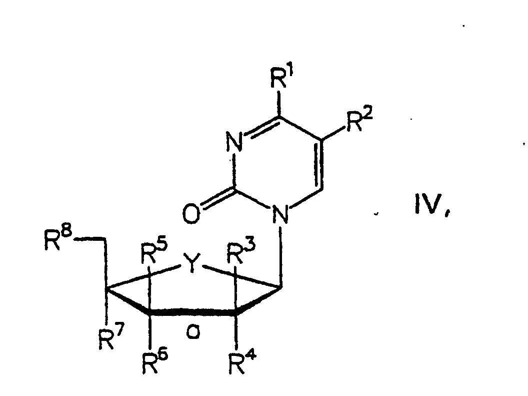 où :
R¹ à R⁸ présentent les valeurs indiquées dans les revendications 4 à 13, R⁸ pouvant en outre être un groupe 4-mono ou 4,4'-diméthoxytriphénylméthoxy, hydrogénophosphonate ou thio-hydrogénophosphonate,
R³, R⁴, R⁵ et R⁶ peuvent aussi désigner un radical carboxylé, linéaire ou ramifié, qui comporte de 1 à 24 atomes de carbone et peut être phényl-substitué, sous la réserve que les radicaux R³, R⁴, R⁵, R⁶ et R⁸ soient un hydrogénophosphonate ou thio-hydrogénophosphonate quand, dans la formule V, X²B est un hydroxyle ou un thiol,
pour terminer par une oxydation,
on traite éventuellement le milieu réactionnel par chromatographie, on convertit éventuellement les groupes 4-mono ou 4,4'-diméthoxytriphénylméthyle en groupes hydroxyle, et le cas échéant, on convertit les radicaux acyle en groupes mercapto, hydroxyle et/ou amino par hydrolyse.
revendications précédentes dans un milieu véhicule ou diluant pharmaceutiquement accceptable, éventuellement en combinaison avec une mileu de formulation pharmaceutiquement acceptable ou incorporé dans un liposome ou une nanoparticule.
